# EUROPÄISCHE PATENTSCHRIFT

(11) **EP 1 456 197 B1**
(45) Veröffentlichungstag und Bekanntmachung des Hinweises auf die Patenterteilung: **28.02.2007**
(21) Anmeldenummer: 02799058.9
(22) Anmeldetag: 18.12.2002
(51) Int. Cl.: C07D 401/04, A61K 31/517, A61P 29/00

(54) **IN 3-POSITION HETEROCYCLISCH SUBSTITUIERTE PIPERIDIN-2,6-DIONE**
PIPERIDINE-2,6-DIONES THAT ARE HETEROCYCLICALLY SUBSTITUTED IN POSITION 3
PIPERIDINE-2,6-DIONES SUBSTITUEES, SUR HETEROCYCLE, EN POSITION 3

(30) Priorität: 21.12.2001 DE 10163595
(43) Veröffentlichungstag der Anmeldung: 15.09.2004
(73) Patentinhaber: Grünenthal GmbH, 52078 Aachen (DE)
(72) Erfinder: BUSCHMANN, Helmut, Heinrich, E-08950 Esplugues de Llobregat (ES); FROSCH, Stefanie, 52078 Aachen (DE); GERMANN, Tieno, 52080 Aachen (DE); ZIMMER, Oswald, Karl, 52146 Würselen (DE); THEIL, Fritz, 12247 Berlin (DE)
(86) Internationale Anmeldenummer: PCT/EP2002/014447
(87) Internationale Veröffentlichungsnummer: WO 2003/053956

(56) Entgegenhaltungen:
- DE-A- 10 002 509
- US-A- 6 080 742

## Beschreibung

Die Erfindung betrifft in 3-Position heterocyclisch substituierte Piperidin-2,6-dione der allgemeinen Formel I 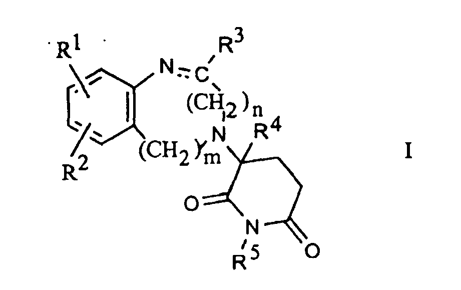 ihre Herstellung sowie ihre Verwendung in Arzneimitteln.

Autoimmunerkrankungen entstehen aufgrund einer Reaktivität des Immunsystems gegen körpereigene Strukturen. Dabei ist die normalerweise vorhandene Toleranz gegenüber körpereigenem Gewebe aufgehoben. In der Pathogenese der verschiedenen Autoimmunerkrankungen spielen neben Antikörpern insbesondere T-Lymphozyten und Monozyten/Makrophagen eine entscheidende Rolle. Aktivierte Monozyten/Makrophagen sezernieren eine Vielzahl verschiedener entzündungsfördernder Mediatoren, die direkt oder indirekt für die Zerstörung der von der Autoimmunerkrankung betroffenen Gewebe verantwortlich sind. Die Aktivierung von Monozyten/Makrophagen erfolgt entweder in der Interaktion mit T-Lymphozyten oder über bakterielle Produkte wie Lipopolysaccharid (LPS). Eine von aktivierten Monozyten/Makrophagen gebildete entzündungsfördernde Substanz ist das Interleukin-12 (IL-12).

IL-12 ist ein heterodimeres Molekül, welches aus einer kovalent verbundenen p35 und p40 Kette besteht. Es wird von antigenpräsentierenden Zellen (Monozyten/Makrophagen, dendritische Zellen, B-Lymphozyten) nach Aktivierung durch verschiedene mikrobielle Produkte wie LPS, Lipopeptide, bakterielle DNA oder in der Interaktion mit aktivierten T-Lymphozyten gebildet (Trinchieri 1995. Ann.Rev.Immunol. 13: 251). IL-12 hat eine zentrale immunregulatorische Bedeutung und ist verantwortlich für die Entwicklung entzündungsfördernder TH1 Reaktivitäten. Beim Vorliegen einer TH1 Immunreaktion gegen Eigenantigene kommt es zum Auftreten schwerer Erkrankungen, wie in zahlreichen tierexperimentellen und ersten klinischen Untersuchungen klar dokumentiert wird. In verschiedenen Tiermodellen für Erkrankungen wie Rheumatoide Arthritis, Multiple Sklerose, Diabetes mellitus sowie entzündliche Darm-, Haut- und Schleimhauterkrankungen zeigt sich die pathophysiologische Bedeutung von IL-12 (Trembleau et al. 1995. Immunol. Today 16: 383; Müller et al. 1995. J.Immunol. 155: 4661; Neurath et al. 1995. J.Exp.Med. 182: 1281; Segal et al. 1998. J.Exp.Med. 187: 537; Powrie et al. 1995. Immunity 3: 171; Rudolphi et al. 1996. Eur.J.Immunol. 26: 1156; Bregenholt et al. 1998. Eur.J.Immunol. 28: 379). Durch Applikation von IL-12 ließ sich die jeweilige Erkrankung auslösen bzw. nach Neutralisierung von endogenem IL-12 zeigte sich ein abgeschwächter Krankheitsverlauf bis hin zu einer Heilung der Tiere. Die Anwendung von Antikörpern gegen IL-12 am Menschen steht derzeit bevor.

Das Zytokin IL-10 inhibiert die Synthese der entzündungsfördernden Zytokine TNFα, IL-1, IL-6, IL-8, IL-12 und GM-CSF durch humane und murine Monozyten/Makrophagen (Fiorentino et al., 1991. J.Immunol. 146: 3444; De Waal Malefyt et al. 1991. J.Exp.Med. 174:1209). Dadurch kommt es indirekt auch zu einer Hemmung der Synthese von IFN-γ durch TH1 Lymphozyten. Interessanterweise tritt die Bildung von IL-10 durch Monozyten/Makrophagen mit einer geringen zeitlichen Verzögerung gegenüber der Synthese der entzündungsfördernden Zytokine auf. Die Behandlung von antigenpräsentierenden Zellen mit IL-10 resultiert in deren Deaktivierung. Solche Zellen sind nicht in der Lage, T-Lymphozyten zur Proliferation bzw. zur Synthese von IFN-γ zu aktivieren. Jedoch sezernieren diese T-Lymphozyten selbst große Mengen an IL-10 und vermögen Entzündungsreaktionen zu unterdrücken, wie am Beispiel eines Tiermodells zu entzündlichen Darmerkrankungen gezeigt werden konnte (Groux et al., 1997. Nature 389: 737). Auch die Entwicklung entzündlicher Hauterkrankungen kann durch IL-10 verhindert werden (Enk et al., 1994. J.Exp.Med. 179: 1397).

Zusammenfassend läßt sich sagen, daß ein Überschuß an IL-12 bzw. ein Mangel an IL-10 die Pathophysiologie einer Vielzahl entzündlicher/Autoimmun-Erkrankungen bedingt. Ansätze zur Wiederherstellung des Gleichgewichts zwischen entzündungsfördernden (IL-12) und entzündungshemmenden (IL-10) Zytokinen haben daher ein großes therapeutisches Potential bei oben genannten Erkrankungen.

IL-12 ist darüber hinaus auch an der Regulation des Überlebens von Zellen beteiligt. Unkontrolliertes Zellwachstum wird u.a. durch Apoptose (programmierter Zelltod) reguliert. An T-Lymphozyten wurde gezeigt, daß IL-12 eine anti-apoptotische Wirkung besitzt und das Überleben von T-Zellen fördert (Clerici et al. 1994. Proc.Natl.Acad.Sci.USA 91: 11811; Estaquier et al. 1995. J.Exp.Med. 182: 1759). Eine lokale Überproduktion von IL-12 kann daher zum Überleben von Tumorzellen beitragen. Inhibitoren der Bildung von IL-12 besitzen daher auch in der Tumortherapie ein großes therapeutisches Potential.

Eine Substanz mit dem immunmodulatorischen Wirkprinzip der IL-12 Hemmung und der IL-10 Steigerung ist Thalidomid. Klinische Studien haben in jüngster Zeit den positiven Einfluß von Thalidomid auf folgende Erkrankungen gezeigt: Erythema nodosum leprosum (Sampaio et al. 1993.

J.Infect.Dis. 168: 408), Aphthosis (Jacobson et al. 1997. N.Engl.J.Med. 336: 1487), chronische Abstoßungsreaktionen (Vogelsang, et al. 1992. N.Engl.J.Med. 326: 1055), entzündliche Darmerkrankungen (Ehrenpreis et al. 1999. Gastroenterology 117: 1271; Vasiliauskas et al. 1999. Gastroenterology 117: 1278) sowie zahlreiche Hauterkrankungen (Bernal et al. 1992. lnt.J.Derm. 31: 599). Klinische Studien laufen derzeit auch zur Therapie für eine Reihe von Tumorerkrankungen (Rajkumar, 2001. Oncology 15: 867). Eine Wirksamkeit bei multiplem Myelom scheint sicher (Singhal, 1999. N.Engl.J.Med. 341: 1565).
Thalidomid löst jedoch auch eine Reihe von Nebenwirkungen aus, zu denen Sedation, Teratogenität und Neuropathie zählen. Ferner ist die Substanz schwer löslich und stark hydrolyseempfindlich.

Die der Erfindung zugrundeliegende Aufgabe bestand deshalb darin, neue Verbindungen zu generieren, die das oben beschriebene immunmodulierende Prinzip besitzen und darüber hinaus eine geringere Hydrolyseempfindlichkeit und bessere Löslichkeit aufweisen.

Diese an die zu generierenden Verbindungen gestellten Anforderungen werden von bestimmten substituierten Piperidin-2,6-dionen erfüllt. Z.B., DE10002509 offenbart in 3-Position Aryl-substituierte Piperidin-2,6-dione.

Gegenstand der Erfindung sind dementsprechend in 3-Position heterocyclisch substituierte Piperidin-2,6-dione der allgemeinen Formel I, 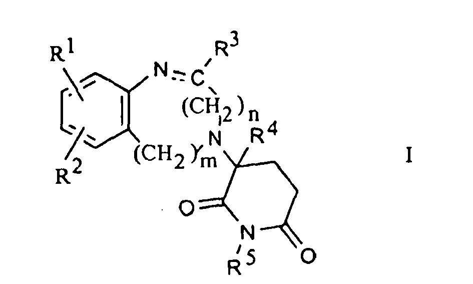 in der
- R¹ und R²,: gleich oder verschieden voneinander, H, Br, Cl, F, CF₃, OH, NO₂, NH₂, N(CH₃)₂, C₁₋₃-Alkyl, C₁₋₃-Alkoxy, Phenyl oder zusammengenommen einen ankondensierten Benzolring bedeuten, wobei die Ringe gegebenenfalls mit R^{1⁻} und/oder R² substituiert und R¹ und R² wie oben definiert sind,
- R³: für H, die Methylgruppe oder, für den Fall, daß eine C-N-Einfachbindung vorliegt, für OH, C₁₋₃-Alkoxy oder eine [O(CO)C₁₋₃-Alkyl]-Gruppe steht, oder zusammen mit dem C-Atom eine Carbonyl-Gruppe darstellt,
- R⁴: H, F, CF₃ oder C₁₋₃-Alkyl bedeutet,
- R⁵: für H, eine CH₂-OH-Gruppe oder einen Rest CH₂-NR⁶R⁷, in dem R⁶ und R⁷ gleich oder verschieden sind und eine Alkylgruppe mit 1-6 C-Atomen (geradkettig oder verzweigt) oder zusammen mit dem N-Atom einen Pyrrolidin-, Piperidin-, Hexamethylenimin- oder Morpholinring bedeuten, steht,
- n: 0 oder 1
- und m: 0, 1 oder 2 bedeutet.

Die erfindungsgemäßen Verbindungen können als reine Enantiomere oder nichtracemische Enantiomerengemische, Racemate, Diastereomere, oder Diastereomerengemische sowohl in Form ihrer freien Basen als auch von Salzen mit physiologisch verträglichen organischen oder anorganischen Säuren vorliegen.

Bevorzugte Verbindungen sind solche, in denen R¹ und R², gleich oder verschieden voneinander, H, Br, Cl, F, CF₃, NO₂, NH₂, C₁₋₃-Alkyl, C₁₋₃-Alkoxy, oder zusammengenommen einen ankondensierten Benzolring bedeuten, R³ für H oder OH, R⁴ für H oder eine Methylgruppe und R⁵ für H oder einen Rest CH₂-NR⁶R⁷ steht, in dem R⁶ und R⁷ zusammen mit dem N-Atom einen Piperidinring bedeutet, und n = 0 und m = 1 oder 2 ist.

Besonders bevorzugt sind Verbindungen, in denen R¹ und R², gleich oder verschieden voneinander, H, Cl, F, CH₃ oder NO₂ bedeuten, R³, R⁴ und R⁵ Wasserstoff bedeuten, n = 0 und m = 1 ist und eine C=N-Doppelbindung vorliegt

Zu weiteren bevorzugten Verbindungen zählen:
3-(7-Chlor-4H-chinazolin-3-yl)piperidin-2,6-dion und das entsprechende Hydrobromid
3-(6-Chlor-4H-chinazolin-3-yl)piperidin-2,6-dion und das entsprechende Hydrobromid
3-(4H-Chinazolin-3-yl)piperidin-2,6-dion und das entsprechende Hydrobromid oder Hydrochlorid
3-(2-Hydroxy-1,4-dihydro-2H-chinazolin-3-yl)piperidin-2,6-dion
3-(6-Methoxy-4H-chinazolin-3-yl)piperidin-2,6-dion und das entsprechende Hydrochlorid.
3-(5-Chlor-4H-chinazolin-3-yl)piperidin-2,6-dion
3-(8-Chlor-4H-chinazolin-3-yl)piperidin-2,6-dion
3-(6-Fluor-4H-chinazolin-3-yl)piperidin-2,6-dion
3-(8-Methoxy-4H-chinazolin-3-yl)piperidin-2,6-dion und das entsprechende Hydrochlorid
3-(7-Fluor-4H-chinazolin-3-yl)piperidin-2,6-dion und das entsprechende Hydrochlorid
3-(4H-Benzo[g]chinazolin-3-yl)piperidin-2,6-dion
3-(5-Fluor-4H-chinazolin-3-yl)piperidin-2,6-dion
3-(5-Nitro-4H-chinazolin-3-yl)piperidin-2,6-dion
3-(7-Trifluormethyl)-4H-chinazolin-3-yl)piperidin-2,6-dion
3-(7-Nitro-4H-chinazolin-3-yl)piperidin-2,6-dion
3-(8-Brom-6-methyl-4H-chinazolin-3-yl)piperidin-2,6-dion
3-(5-Methyl-4H-chinazolin-3-yl)piperidin-2,6-dion
3-(6,7-Difluor-4H-chinazolin-3-yl)piperidin-2,6-dion
3-Methyl-3-(4H-chinazolin-3-yl)piperidin-2,6-dion
3-(4,5-Dihydrobenzo[d][1,3]diazepin-3-yl)piperidin-2,6-dion
3-(5-Amino-4H-chinazolin-3-yl)piperidin-2,6-dion und das entsprechende Dihydrochlorid
3-(7-Amino-4H-chinazolin-3-yl)piperidin-2,6-dion und das entsprechende Dihydrochlorid
3-(7-Fluor-4H-chinazolin-3-yl)1-piperidin-1-ylmethyl-piperidin-2,6-dion

Ganz besonders bevorzugt sind davon die Verbindungen :
3-(7-Chlor-4H-chinazolin-3-yl)piperidin-2,6-dion und das entsprechende Hydrobromid
3-(4H-Chinazolin-3-yl)piperidin-2,6-dion und das entsprechende Hydrobromid
3-(5-Chlor-4H-chinazolin-3-yl)piperidin-2,6-dion
3-(7-Fluor-4H-chinazolin-3-yl)piperidin-2,6-dion und das entsprechende Hydrochlorid
3-(5-Fluor-4H-chinazolin-3-yl)piperidin-2,6-dion.

Ein weiterer Gegenstand der vorliegenden Erfindung sind Verfahren zur Herstellung der erfindungsgemäßen Verbindungen der Formel I

Die Verbindungen der Formel I werden dadurch erhalten, daß man zunächst eine Aminoverbindung der allgemeinen Formel II 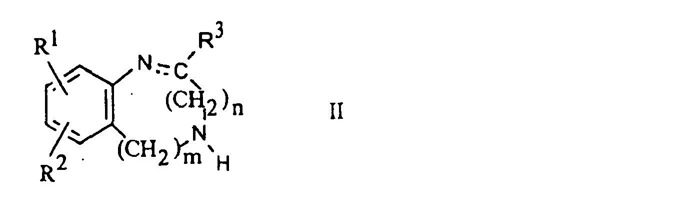 mit einem 3-Brompiperidin-2,6-dionderivat der allgemeinen Formel III 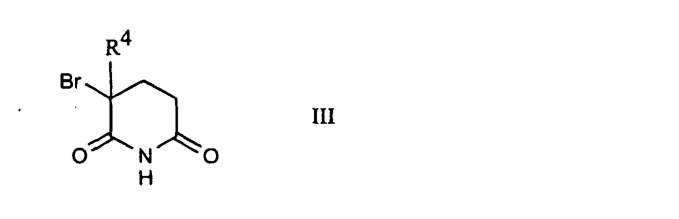 zu einer Verbindung der allgemeinen Formel IA alkyliert, 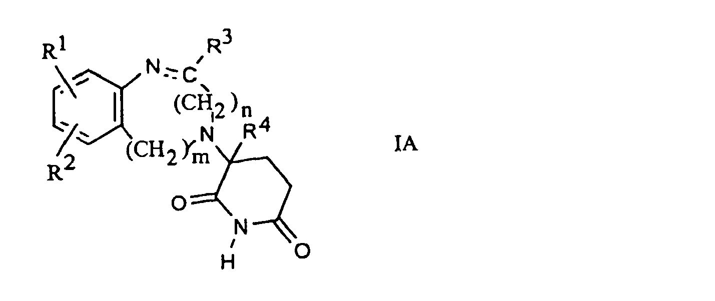 wobei in den Verbindungen IA, II und III die Reste R¹ bis R⁴ wie oben definiert sind, und anschließend, wenn R⁵ nicht für Wasserstoff stehen soll, diesen Rest durch Umsetzung mit Formaldehyd, gegebenenfalls zusammen mit einem Amin der allgemeinen Formel HNR⁶R⁷, in der R⁶ und R⁷ wie oben definiert sind, einführt.
Steht in der Verbindung der Formel IA der Rest R⁴ für Wasserstoff, so läßt sich dieser durch an sich bekannte Alkylierungs- oder Halogenierungsreaktionen gegen die übrigen definitionsgemäßen R⁴-Substituenten austauschen.

Stehen in der Verbindung der Formel IA R¹ und/oder R² für eine Nitrogruppe, so lassen sich daraus in an sich bekannter Weise, z.B. durch Reduktion mit katalytisch angeregtem Wasserstoff, Verbindungen IA herstellen, in denen R¹ und/oder R² die Aminogruppe bedeuten.

Verbindungen der Formel I werden auch dadurch erhalten, daß man eine Aminoverbindung der Formel II zunächst mit einem 3-Brompiperidin-2-onderivat der Formel IV 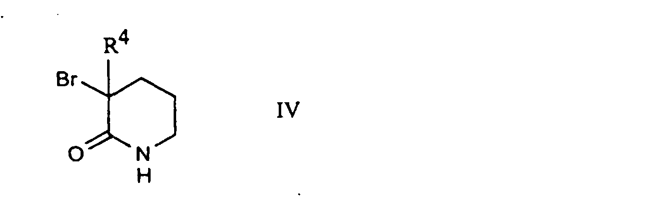 zu einer Verbindung der allgemeinen Formel IB alkyliert, 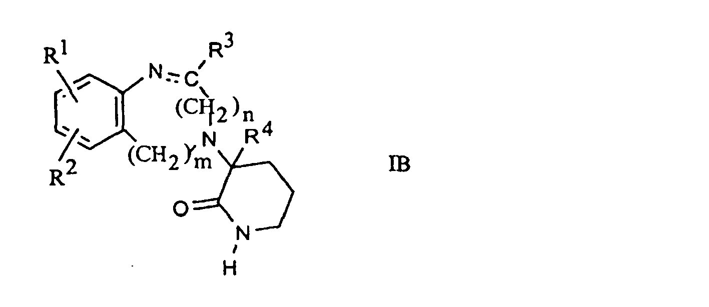 diese, vorzugsweise mit m-Chlor-perbenzoesäure oder Ruthenium (IV)-oxid/Natriumperiodat, zu einer Verbindung der oben genannten Formel IA oxidiert und gegebenenfalls andere Reste R⁴ und/oder den Rest R⁵ einführt.

Bevorzugte Verbindungen der Formel I, in denen m = 1 und n = 0 bedeuten und R³ für H oder OH steht, werden hergestellt, indem ein Formamidderivat der allgemeinen Formel VI, 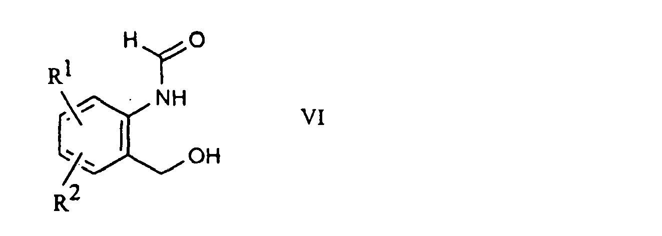 in der R¹ und R² wie oben definiert sind, und das durch selektive N-Formylierung des entsprechenden 2-Aminobenzylalkohols oder durch selektive O-Entformylierung des N,O-Bisformylderivats, zum Beispiel enzymatisch mit Hilfe von CAL-B, zugänglich ist, zunächst in an sich bekannter Weise, z.B. mit Pyridiniumdichromat, zu einem Benzaldehyd der allgemeinen Formel VII oxidiert, 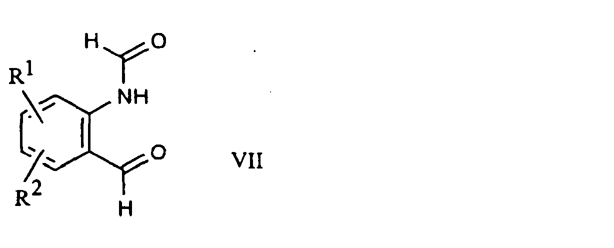 der durch reduktive Aminierung mit Glutamin unter Einsatz von komplexen Borhydriden, wie z.B. Natriumborhydrid, in Verbindungen der allgemeinen Formel VIII übergeführt wird.

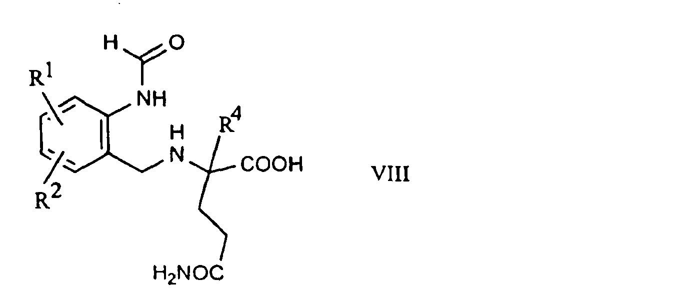

Diese Verbindungen der allgemeinen Formel VIII lassen sich dann, z.B. mit N,N'-Carbonyldiimidazol, zu Glutarimiden der allgemeinen Formel IX cyclisieren, vorzugsweise nach vorherigem Schutz der Aminfunktion durch z.B. die Benzyloxycarbonylgruppe, die anschließend, z.B. mit Bromwasserstoff in Eisessig, wieder abgespalten wird.

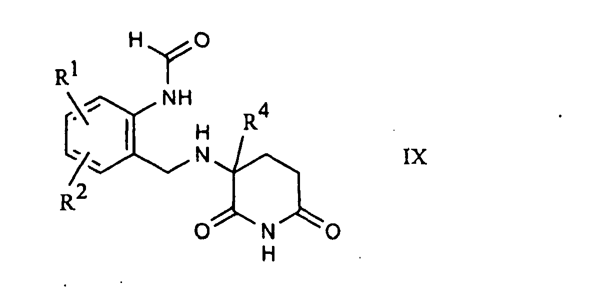

Aus den Verbindungen der allgemeinen Formel IX werden in protischen Lösungsmitteln wie z.B. Wasser unter Säurekatalyse schließlich Verbindungen der allgemeinen Formel I erhalten, in denen R¹, R² und R⁴ wie oben definiert sind, m für 1, n für 0 steht, eine C=N-Doppelbindung vorliegt und R³ bzw. R⁵ ein Wasserstoffatom darstellen, das sich im Falle von R⁵ wie oben beschrieben gegen die übrigen definitionsgemäßen Substituenten austauschen läßt.

Wird in den Verbindungen der allgemeinen Formel IX, in denen die Aminfunktion durch den Benzyloxycarbonylrest geschützt ist, dieser hydrogenolytisch abgespalten, so werden Verbindungen der allgemeinen Formel I erhalten, in denen R¹ und R² wie oben definiert sind, eine C-N-Einfachbindung vorliegt und R³ für die Hydroxygruppe steht. Mit verdünnten Säuren in organischen Lösungsmitteln wie z.B. Methanol lassen sich diese durch Wasserabspaltung in Verbindungen der allgemeinen Formel I mit C=N-Doppelbindung und mit R³ = Wasserstoff umwandeln.

Wird nach reduktiver Aminierung der Verbindungen der allgemeinen Formel VII das Reaktionsgemisch mit Säuren behandelt, entstehen daraus Verbindungen der allgemeinen Formel X, in denen R¹, R² und R⁴ wie oben definiert sind, 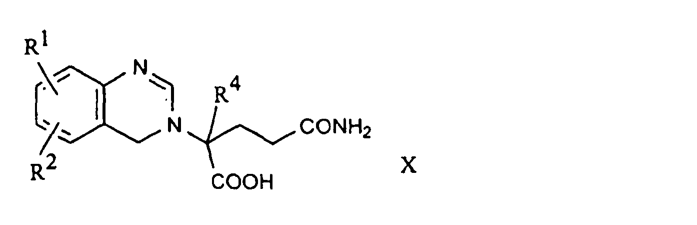 die sich durch Cyclisierung, z.B. mit Essigsäureanhydrid/Acetylchlorid, in Verbindungen der allgemeinen Formel I, in denen m = 1 und n = 0 bedeuten, eine C=N-Doppelbindung vorliegt, R¹, R² und R⁴ wie oben definiert sind und R³ bzw. R⁵ für Wasserstoff stehen, überführen lassen. Danach werden gegebenenfalls andere definitionsgemäße Reste R⁴ und /oder R⁵ wie oben beschrieben eingeführt.

In analoger Weise lassen sich damit auch Verbindungen der Formel I erhalten, in denen m = 2 bedeutet und die übrigen Reste wie oben definiert sind.

Die erfindungsgemäßen Verbindungen besitzen immunmodulatorische Aktivität, sie induzieren eine drastische Reduktion der IL-12 Produktion in LPS-aktivierten Monozyten bei gleichzeitiger Steigerung der IL-10 Produktion. Aufgrund dieses Wirkprinzips haben diese Verbindungen ein großes therapeutisches Potential bei Krankheiten, wo eine überschießende IL-12 Produktion und ein relativer Mangel an IL-10 für die Pathogenese verantwortlich gemacht wird, das heißt, solche Verbindungen sind zur Behandlung und/oder Prophylaxe von Entzündungs- und Autoimmunerkrankungen zu nutzen. Aufgrund der anti-apoptotischen Wirkung von IL-12 sind die erfindungsgemäßen Verbindungen auch zur Suppression der Bildung von IL-12 bei hämatologisch-onkologischen Erkrankungen geeignet.

Dies unterscheidet die beanspruchten Verbindungen von bekannten Immunmodulatoren wie Corticosteroiden (z.B. Dexamethason), die sowohl die Synthese von IL-12 als auch die von IL-10 durch Monozyten supprimieren.

Gegenüber Thalidomid zeichnen sich die neuen Verbindungen überraschenderweise durch eine bessere Wirksamkeit, eine gute Wasserlöslichkeit sowie eine geringere Hydrolyseempfindlichkeit aus.

Zu den Erkrankungen oben genannter Formenkreise zählen unter anderem Entzündungen der Haut (z.B. atopische Dermatitis, Psoriasis, Ekzeme, Erythema nodosum leprosum), Entzündungen der Atemwege (z.B. Bronchitis, Pneumonie, Asthma bronchiale, ARDS (adult respiratory distress syndrome), Sarkoidose, Silikose/Fibrose), Entzündungen des Gastrointestinaltraktes (z.B. gastroduodenale Ulcera, Morbus Crohn, ulcerative Colitis), ferner Erkrankungen wie Hepatitis, Pankreatitis, Appendizitis, Peritonitis, Nephritis, Aphthosis, Konjunktivitis, Keratitis, Uveitis, Rhinitis.
Die Autoimmunerkrankungen umfassen z.B. Erkrankungen des arthritischen Formenkreises (z.B. rheumatoide Arthritis, HLA-B27 assoziierte Erkrankungen, rheumatoide Spondylitis), ferner Multiple Sklerose, jugendlicher Diabetes oder Lupus erythematosus.
Weitere Indikationen sind Sepsis, septischer Schock, bakterielle Meningitis, mycobakterielle Infektionen, opportunistische Infektionen bei AIDS, Kachexie, Transplantat-Abstoßungs-Reaktionen, Graft-versus-Host Reaktionen sowie chronisches Herzversagen, Herzinsuffizienz, das Reperfusions-Syndrom sowie Atherosklerose.
Darüber hinaus zählen auch chronische Schmerzzustände, Fibromyalgie, Morbus Sudeck (reflex sympathetic dystrophy (RSD)) zu den Indikationen.

Ferner gehören hämatologische Erkrankungen wie multiples Myelom, Myelodisplastisches Syndrom und Leukämien sowie weitere onkologische Erkrankungen wie z.B. Glioblastom, Prostata-, Nierenzell-, Mamma-, Schilddrüsen-, Kopf- und Hals-, Pancreas- und colorectales Carcinom sowie Melanom und Kaposi's Sarcom zu den Krankheitsbildern, bei denen die beschriebenen Immunmodulatoren einzusetzen sind.

Erfindungsgemäße Arzneimittel enthalten neben mindestens einer Verbindung der allgemeinen Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen hängen davon ab, ob das Arzneimittel oral, rektal, ophthalmisch (intravitreal, intracameral), nasal, topisch (einschließlich buccal und sublingual), vaginal oder parenteral (einschließlich subkutan, intramuskulär, intravenös, intradermal, intratracheal und epidural) verabreicht werden soll.

Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trockenzubereitungen sowie Sprays. Kutane Applikationsformen sind Salben, Gele, Cremes und Pasten. Ophthalmische Applikationsformen umfassen Tropfen, Salben und Gele. Erfindungsgemäße Verbindungen in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an Patienten zu verabreichende Wirkstoffmenge variiert in Abhängigkeit vom Gewicht des Patienten, von der Applikationsart, der Indikation und dem Schweregrad der Erkrankung.

### Beispiele

Die folgenden Beispiele dienen zur näheren Erläuterung der vorliegenden Erfindung.
Als stationäre Phase für die chromatographischen Trennungen wurde Kieselgel 60 (0,040 bis 0,063 mm) der Firma E. Merck, Darmstadt, eingesetzt. Die Mischungsverhältnisse der Elutionsmittel sind stets in Volumen/Volumen angegeben.
Die Substanzen wurden über ihren Schmelzpunkt und/oder das ¹H-NMR-Spektrum charakterisiert. Die Aufnahme der Spektren erfolgte bei 300 MHz mit dem Gerät Gemini 300 der Firma Varian. Die chemischen Verschiebungen sind in ppm angegeben (δ-Skala). Als interner Standard wurde Tetramethylsilan (TMS) verwendet.

### Beispiel 1

### 3-(7-Chlor-4H-chinazolin-3-yl)piperidin-2,6-dion; Hydrobromid

### Stufe 1:

### N-(5-Chlor-2-hydroxymethlyphenyl)formamid

Eine Lösung von 5,00 g (2-Amino-4-chlorphenyl) methanol, 2,73 g Ameisensäurecyanmethylester und 0,04 g 4-N,N-Dimethylaminopyridin in 50 ml wasserfreiem Tetrahydrofuran wurde 72 Stunden bei 20°C gerührt. Dann wurde das Lösungsmittel im Vakuum abgedampft und der Rückstand in 50 ml Essigsäureethylester aufgenommen. Die Lösung wurde nacheinander zweimal mit je 100 ml 0,01 N Salzsäure und gesättigter Natriumchloridlösung gewaschen, über Natriumsulfat getrocknet und dann im Vakuum eingedampft. Der Rückstand wurde durch Flashchromatographie an Kieselgel mit Essigsäureethylester/Cyclohexan (2/1) als Elutionsmittel gereinigt. Man erhielt so 1,41 g (24 % der Theorie) der Titelverbindung in Form leicht gelblicher Kristalle.
Schmelzpunkt: 120 - 124°C

### Stufe 2:

### N-(5-Chlor-2-formylphenyl)formamid

Eine Mischung aus 1,40 g des Produkts aus Stufe 1 und 3,40 g Pyridiniumdichromat in 350 ml wasserfreiem Dichlormethan wurde 24 Stunden bei 20°C gerührt. Dann wurde filtriert und das Filtrat im Vakuum eingedampft. Der Rückstand wurde durch Flashchromatographie an Kieselgel mit Essigsäureethylester/Cyclohexan (1/1) als Elutionsmittel gereinigt, wobei 0,88 g (64 % der Theorie) der Titelverbindung in kristalliner Form anfielen.
Schmelzpunkt: 131 - 136°C

### Stufe 3:

### 2-[Benzyloxycarbonyl-4-chlor-2-formylaminobenzyl)amino]-4-carbamoylbuttersäure

Eine Lösung von 0,75 g L-Glutamin in 2,8 ml 2 N Natronlauge wurde mit einer Lösung von 1,02 g des Produkts aus Stufe 2 in 12 ml Tetrahydrofuran versetzt. Man rührte die Mischung 1 Stunde bei 20°C, kühlte auf 0°C und gab portionsweise 0,13 g Natriumborhydrid hinzu. Nach 12 Stunden Rühren bei 20°C wurde innerhalb 1 Stunde eine Lösung von 1,47 g Pyrokohlensäuredibenzylester in 5,5 ml Tetrahydrofuran zugetropft; anschließend wurde mit 2 ml 2 N Natronlauge versetzt. Nach 12 Stunden Rühren bei 20°C wurde Tetrahydrofuran im Vakuum weitgehend abgedampft und der Rückstand dreimal mit je 20 ml Diethylether extrahiert. Die wässrige Phase wurde mit 2 N Salzsäure auf pH 1 gebracht und dreimal mit je 20 ml Essigsäureethylester extrahiert. Die vereinigten organischen Phasen wurden über Natriumsulfat getrocknet und im Vakuum eingedampft, wobei 0,99 g (40 % der Theorie) der Titelverbindung anfielen, die ohne Reinigung weiter umgesetzt wurden.

### Stufe 4:

### (4-Chlor-2-formylaminobenzyl)-(2,6-dioxopiperidin-3-yl-) carbamidsäurebenzylester

Eine Lösung von 0,98 g des Produkts aus Stufe 3 und 0,97 g N,N'-Carbonyldiimidazol in 50 ml wasserfreiem Tetrahydrofuran wurde 8 Stunden zum Rückfluß erhitzt. Nach dem Abkühlen wurde im Vakuum eingedampft, der Rückstand in je 40 ml Wasser bzw. Essigsäureethylester aufgenommen und die organische Phase abgetrennt. Die wässrige Phase wurde dreimal mit je 20 ml Essigsäureethylester extrahiert. Die vereinigten organischen Phasen wurden dreimal mit je 20 ml Wasser, dann mit 20 ml gesättigter Natriumchloridlösung gewaschen, über Natriumsulfat getrocknet und im Vakuum eingedampft. Nach Reinigung des Rückstands durch Flashchromatographie an Kieselgel mit Essigsäureethylester/Cyclohexan (1/1) blieben 0,37 g (16 % der Theorie, bezogen auf das Produkt der Stufe 2) der Titelverbindung zurück.

### Stufe 5:

### N-{5-Chlor-2-[(2,6-dioxopiperidin-3-ylamino)methyl]phenyl}formamid; Hydrobromid

Eine Lösung von 0,38 g des Produkts aus Stufe 4 in 2 ml Essigsäure wurde mit 0,9 ml Bromwasserstoff in Essigsäure (33 % HBr) versetzt und die Mischung 1 Stunde bei 20°C gerührt. Dann wurde auf 400 ml Diethylether gegossen, auf 0°C abgekühlt und der ausgefallene Feststoff abgetrennt. Dieser wurde mehrfach mit Diethylether gewaschen und im Vakuum getrocknet, wobei 0,27 g (83 % der Theorie) der Titelverbindung in Form farbloser Kristalle anfielen.
Schmelzpunkt: 155 - 180°C (Zersetzung)

### Stufe 6:

### 3-(7-Chlor-4H-chinazolin-3-yl)piperidin-2,6-dion; Hydrobromid

Eine Lösung von 0,20 g des Produkts aus Stufe 5 in 10 ml destilliertem Wasser wurde 72 Stunden bei 20°C gerührt. Dann wurde filtriert, das Filtrat im Vakuum eingedampft und der Rückstand getrocknet. Dabei fielen 0,13 g (68 % der Theorie) der Titelverbindung als weißer Feststoff an. Schmelzpunkt: 160 - 200 °C (Zersetzung)
- 1H-NMR (DMSO-d₆):: 2,57 (m, 2H); 2,82 (m, 2H); 4,80 (m, 1 H); 4,95 (m, 1 H); 5,10 (m, 1 H) 7,30 (m, 2H); 7,45 (m, 1 H); 8,68 (s, 1 H); 11,42(s, 1 H)

### Beispiel 2

### 3-(6-Chlor-4H-chinazolin-3-yl)piperidin-2,6-dion; Hydrobromid

Bei Ersatz des in Beispiel 1, Stufe 1, verwendeten Benzylalkohols durch das 5-Chlor-isomer und Anwendung der in den Stufen 1 bis 6 beschriebenen Verfahrensweise erhielt man die Titelverbindung in Form leicht gelber Kristalle.
- 1H-NMR (DMSO-d₆):: 2,19 (m, 1 H); 2,43 (m, 1 H); 2,75 (m, 2H); 4,78 (m, 2H); 5,02 (m, 1H); 7,18 (d, 1 H); 7,38 (d, 1H); 7,45 (q, 1 H); 8,59 (s, 1 H); 11,35 (s,1H)

### Beispiel 3

### 3-(4H-Chinazolin-3-yl)piperidin-2,6-dion; Hydrobromid

### Stufe 1:

### (2-Formylaminobenzyl)-(2,6-dioxopiperidin-3-yl) carbamidsäurebenzylester

Unter Einsatz von (2-Aminophenyl)methanol in Beispiel 1, Stufe 1 und Anwendung der in den Stufen 1 bis 4 beschriebenen Verfahrensweise erhielt man die Titelverbindung in Form eines gelben Pulvers.

### Stufe 2:

### N-{2-[(2,6-Dioxopiperidin-3-ylamino)methyl]phenyl} formamid; Hydrobromid

1,00 g des Produkts aus Stufe 1 wurden wie in Beispiel 1, Stufe 5, beschrieben mit 3 ml Bromwasserstoff in Essigsäure (33 % HBr) umgesetzt. Nach analoger Aufarbeitung wurden 0,79 g (91 % der Theorie) der Titelverbindung in Form eines gelblichen Feststoffes erhalten, der ohne Reinigung weiter umgesetzt wurde.

### Stufe 3:

### 3-(4H-Chinazolin-3-yl)piperidin-2,6-dion; Hydrobromid

Eine Lösung von 0,91 g des Produkts aus Stufe 2 in 5 ml destilliertem Wasser wurde 24 Stunden bei 20°C gerührt. Danach wurde die Reaktionslösung im Vakuum zur Trockene eingedampft. Der zurückbleibende Feststoff wurde mit Diethylether verrieben, wobei 0,85 g (99 % der Theorie) der Titelverbindung als gelbes Pulver zurückblieben.
Schmelzpunkt: 218 - 222°C (Zersetzung)
- ¹H-NMR (DMSO-d₆):: 2,23 (m, 1H); 2,48 (m, 1H); 2,71 (m, 2H); 4,70 (d, 1H); 4,86 (d, 1 H); 4,98 (m, 1 H); 7,22 (m, 2H); 7,34 (m, 2H); 8,53 (s, 1H); 11,35 (s, 1H).

### Beispiel 4

### 3-(2-Hydroxy-1,4-dihydro-2H-chinazolin-3-yl)piperidin-2,6-dion

15,90 g des Produkts aus Beispiel 3, Stufe 1, gelöst in 950 ml Tetrahydrofuran, wurden über 15g Palladium auf Aktivkohle (10 % Pd) bei 20°C unter Normaldruck hydriert. Nach Abtrennen des Katalysators durch Filtration wurde das Filtrat im Vakuum eingedampft. Dabei blieben 10,30 g (98 % der Theorie) der Titelverbindung als Diastereomerengemisch in Form eines weißen Feststoffes zurück.
Schmelzpunkt: 185 - 188°C

### Beispiel 5

### 3-(4H-Chinazolin-3-yl)piperidin-2,6-dion; Hydrochlorid

Eine Lösung von 10,20 g des Produkts aus Beispiel 4 in 210 ml Methanol und 3,2 ml 12 N Salzsäure wurde 24 Stunden bei 20°C gerührt. Dann wurde im Vakuum weitgehend eingedampft, wobei die Titelverbindung in kristalliner Form anfiel, von der nach Abtrennen durch Filtration und Trocknen 9,00 g (82 % der Theorie) zurückblieben.
Schmelzpunkt: 172 - 178°C
- ¹H-NMR(DMSO-d₆):: 2,22 (m, 1H); 2,48 (m, 1H); 2,71 (m, 2H); 4,70 (d, 1H); 4,86 (d, 1 H); 4,98 (m, 1 H); 7,22 (m, 2H); 7,35 (m, 2H); 8,53 (s, 1H); 11,35 (s, 1H).

### Beispiel 6

### 3-(6-Methoxy-4H-chinazolin-3-yl)piperidin-2,6-dion, Hydrochlorid

### Stufe 1:

### Ameisensäure(2-formylamino-5-methoxybenzyl)ester

Eine Lösung von 7,00 g (2-Amino-5-methoxyphenyl)methanol, 9,8 ml Ameisensäurecyan-methylester und 0,05 g 4-N,N,-(Dimethylamino)pyridin in 30 ml wasserfreiem Tetrahydrofuran wurde 5 Stunden unter Rückfluss erhitzt. Dann wurde im Vakuum eingedampft und der Rückstand in 100 ml Essigsäureethylester aufgenommen. Beim Abkühlen der Lösung auf 0°C bildeten sich Kristalle, die abgesaugt, mit Methanol gewaschen und im Vakuum getrocknet wurden. Man erhielt so 7,20 g (75 % der Theorie) der Titelverbindung.
Schmelzpunkt: 117°C

### Stufe 2:

### N-(2-Hydroxymethyl-4-methoxyphenyl)formamid

Eine Lösung von 5,90 g des Produkts aus Stufe 1 in einem Gemisch aus 190 ml wasserfreiem Acetonitril und 10,3 ml n-Butanol wurde mit 1,70 g Candida antartica B Lipase (CAL-B) versetzt und 65 Stunden bei 20°C gerührt. Dann wurde filtriert und mit Acetonitril gewaschen. Nach Eindampfen des Filtrats im Vakuum blieben 5,00 g (98 % der Theorie) der Titelverbindung zurück.
Schmelzpunkt: 97 - 98°C

### Stufe 3:

### N-(2-Formyl-4-methoxyphenyl)formamid

5,00 g des Produkts aus Stufe 2 wurden wie in Beispiel 1, Stufe 2, beschrieben mit Pyridiniumdichromat oxidiert, wobei 3,62 g (72 % der Theorie) der Titelverbindung anfielen.
Schmelzpunkt: 125 -127°C

### Stufe 4:

### 4-Carbamoyl-2-(6-methoxy-4H-Chinazolin-3-yl)butansäure

Zu einer Lösung von 0,58 g L-Glutamin in 10 ml Methanol und 2 ml 2N Natronlauge wurde eine Lösung von 0,79 g des Produkts aus Stufe 3 in 30 ml Methanol gegeben. Nach 1 Stunde Rühren bei 20°C wurde auf 0°C gekühlt und portionsweise innerhalb von 30 Minuten mit 0,103 g Natriumborhydrid versetzt. Es wurde 12 Stunden bei 0°C gerührt, dann auf pH 2 bis 3 gebracht und weitere 4 Stunden bei 20°C gerührt. Nach Neutralisation der Lösung mit Natronlauge wurde das Methanol abdestilliert. Der wässrige Rückstand wurde zweimal mit je 15 ml Diethylether gewaschen, dann im Vakuum eingedampft. Der Rückstand wurde in 25 ml Methanol aufgenommen und durch Filtration von unlöslichen Bestandteilen befreit. Der durch Eindampfen des Filtrats im Vakuum erhaltene Rückstand wurde ohne Reinigung direkt wie in Stufe 5 beschrieben weiter umgesetzt.

### Stufe 5:

### 3-(6-Methoxy-4H-chinazolin-3-yl)piperidin-2,6-dion; Hydrochlorid

Eine Lösung von 1,20 g des rohen Produkts aus Stufe 4 in je 7 ml Essigsäureanhydrid und Acetylchlorid wurde 5 Stunden bei 70°C gerührt, dann im Vakuum eingedampft. Der Rückstand wurde in 20 ml Methanol aufgenommen und 1 Stunde mit Ionenaustauscher Amberlyst A-21 gerührt. Nach Filtration wurde das Filtrat im Vakuum eingedampft und der Rückstand durch Flashchromatographie mit Chloroform/Methanol (4/1) als Elutionsmittel gereinigt. Die so erhaltene freie Base der Titelverbindung wurde in 4 ml Methanol gelöst und die Lösung mit 0,5 ml einer gesättigten Lösung von HCl-Gas in Diethylether, dann mit 150 ml Diethylether versetzt. Das entstandene Salz wurde abgetrennt und im Vakuum getrocknet, wobei 0,29 g (23 % der Theorie bezogen auf L-Glutamin in Stufe 4) der Titelverbindung anfielen.
- 1H-NMR (DMSO-d₆):: 2,45 (m, 4H); 3,76 (s, 3H); 4,65 (d, 1H); 4,81 (d, 1H); 4,98 (m, 1H); 6,88 (m, 2H); 7,20 (m, 1H); 8,46 (5, 1H); 11,32 (s, 1H)

### Beispiel 7

Unter Einsatz entsprechend substituierter Edukte und unter Anwendung der in Beispiel 6 beschriebenen Verfahrensweise wurden analog erhalten (zum Teil ohne Überführung ins Hydrochlorid):
a) 3-(5-Chlor-4H-chinazolin-3-yl)piperidin-2,6-dion
   Schmelzpunkt: 245 - 250°C (Zersetzung)
b) 3-(8-Chlor-4H-chinazolin-3-yl)piperidin-2,6-dion
   Schmelzpunkt: 238 - 248°C
c) 3-(6-Fluor-4H-chinazolin-3-yl)piperidin-2,6-dion
   Schmelzpunkt: 75 - 79°C
d) 3-(8-Methoxy-4H-chinazolin-3-yl)piperidin-2,6-dion; Hydrochlorid
   Schmelzpunkt: 152 - 159°C
e) 3-(7-Fluor-4H-chinazolin-3-yl)piperidin-2,6-dion
   Schmelzpunkt: 239 - 241°C (Zersetzung)
f) 3-(7-Fluor-4H-chinazolin-3-yl)piperidin-2,6-dion; Hydrochlorid
   Schmelzpunkt: 205 - 207°C
g) 3-(4H-Benzo[g]chinazolin-3-yl)piperidin-2,6-dion
   Schmelzpunkt: 238 - 242°C
h) 3-(5-Fluor-4H-chinazolin-3-yl)piperidin-2,6-dion
   Schmelzpunkt: 237 - 239°C (Zersetzung)
i) 3-(5-Nitro-4H-chinazolin-3-yl)piperidin-2,6-dion
   Schmelzpunkt: 197 - 215°C
j) 3-(7-Trifluormethyl)-4H-chinazolin-3-yl)piperidin-2,6-dion
   Schmelzpunkt: 230 - 234°
k) 3-(7-Nitro-4H-chinazolin-3-yl)piperidin-2,6-dion
   Schmelzpunkt: > 360°C
I) 3-(8-Brom-6-methyl-4H-chinazolin-3-yl)piperidin-2,6-dion
   Schmelzpunkt: 258 - 265°C
m) 3-(5-Methyl-4H-chinazolin-3-yl)piperidin-2,6-dion
   Schmelzpunkt: 217 - 227°C
n) 3-(6,7-Difluor-4H-chinazolin-3-yl)piperidin-2,6-dion
   Schmelzpunkt: ab 360°C (Zersetzung)
o) 3-Methyl-3-(4H-chinazolin-3-yl)piperidin-2,6-dion
   - 1H-NMR (DMSO-d₆):: 1,59 (s, 3H); 1,93 - 2,70 (m, 4H); 4,35 (d, 1H); 4,47 (d, 1 H); 6,90 - 7,15 (m, 4H); 7,36 (s, 1H); 11,04 (s, 1H).

### Beispiel 8

### 3-(4,5-Dihydrobenzo[d][1,3]diazepin-3-yl)piperidin-2,6-dion

### Stufe 1:

### N-[2-(2-Hydroxyethyl)phenyl]formamid

12,6 ml Ameisensäure wurden bei 0°C zu 15 ml Essigsäureanhydrid getropft; die Mischung wurde anschließend 2 Stunden bei 60°C gerührt. Nach Abkühlen auf 20°C wurde mit 140 ml Tetrahydrofuran verdünnt, auf - 4°C abgekühlt und bei dieser Temperatur tropfenweise mit einer Lösung von 18,4 g 2-(2-Aminophenyl)ethanol in 185 ml Tetrahydrofuran versetzt. Nach dreistündigem Rühren bei - 6°C wurde mit wässriger Kaliumhydrogencarbonatlösung (25 % KHCO₃) neutralisiert und viermal mit je 300 ml Essigsäureethylester extrahiert. Die vereinigten Extrakte wurden über Natriumsulfat getrocknet und im Vakuum eingedampft. Dabei fielen 18,4 g (94 % der Theorie) der Titelverbindung in Form gelblicher Kristalle an, die bei 49 - 54°C schmolzen.

### Stufe 2:

### N-[2-(2-Oxoethyl)phenyl]formamid bzw. 2-Hydroxy-2,3-dihydroindol-1-carbaldehyd

Eine Lösung von 18,2 g des Produkts aus Stufe 1 in 270 ml wasserfreiem Dichlorethan wurde bei 0°C unter Rühren portionsweise mit 49,4 g Pyridiniumdichromat versetzt und die Mischung dann 24 Stunden bei 40°C gerührt. Nach Filtration über Celite wurde das Filtrat im Vakuum eingedampft und der Rückstand durch Flashchromatographie an Kieselgel mit Essigsäureethylester/Cyclohexan (1/1) als Elutionsmittel gereinigt. Dabei fielen 7,5 g (42 % der Theorie) der Titelverbindung als gelblicher Feststoff an, der bei 84 - 91 °C unter Zersetzung schmolz.

### Stufe 3:

### 4-Carbamoyl-2-(4,5-dihydrobenzo[d][1,3]diazepin-3-yl)buttersäure

Eine Lösung von 4,1 g L-Glutamin in 14 ml 2N Natronlauge und 7 ml Methanol wurde mit einer Lösung von 4,6 g des Produkts aus Stufe 2 in 110 ml Methanol versetzt und die Mischung 2 Stunden bei 20°C gerührt. Dann wurde auf 0°C abgekühlt, portionsweise mit 0,64 g Natriumborhydrid versetzt und 12 Stunden bei 0°C nachgerührt. Die Reaktionslösung wurde mit 2 N Salzsäure auf einen pH-Wert von 3,5 eingestellt und 20 Stunden bei 20°C gerührt. Nach Neutralisation mit Natronlauge wurde der entstandene Feststoff durch Filtration abgetrennt und das Filtrat im Vakuum vom Methanol befreit. Aus dem wässrigen Rückstand konnten 3,0 g (39 % der Theorie) der rohen Titelverbindung isoliert werden, die ohne Reinigung für die weitere Umsetzung verwendet wurden.

### Stufe 4:

### 3-(4,5-Dihydrobenzo[1][1,3]diazepin-3-yl)piperidin-2,6-dion

3,0 g des Produkts aus Stufe 3 in 12 ml Essigsäureanhydrid wurden mit 12 ml Acetylchlorid versetzt. Das Gemisch wurde 32 Stunden unter Rückfluss und 66 Stunden bei 50°C gerührt, dann wurde im Vakuum eingedampft. Der Rückstand wurde durch Flashchromatographie an Kieselgel mit Trichlormethan/Methanol (4/1) als Elutionsmittel gereinigt, wobei 0,79 g (28 % der Theorie) der Titelverbindung als gelblicher Feststoff anfielen, der ab 250°C unter Zersetzung schmolz.
- 1H-NMR (DMSO-d₆/D₂O):: 2,10 - 2,20 (m, 1H); 2,25 - 2,40 (m, 1H); 2,60 - 2,75 (m, 2H); 3,10 - 3,14 (m, 2H); 3,54 - 3,85 (m, 2H); 4,89 (dd, 1 H); 7,10 - 7,30 (m, 4H); 7,77 (s, 1H).

### Beispiel 9

### a) 3-(5-Amino-4H-chinazolin-3-yl)piperidin-2,6-dion; Dihydrochlorid

Eine Lösung von 0,315 g des Produkts aus Beispiel 7i in 11 ml N,N-Dimethylformamid und 1,1 ml 2N Salzsäure wurde über 0,057 g Platindioxid (80 %) bei 20°C unter einem Wasserstoffdruck von 3 bar katalytisch hydriert. Dann wurde der Katalysator durch Filtration abgetrennt und das Filtrat im Vakuum eingedampft. Der zurückbleibende Feststoff wurde mehrmals mit Toluol bzw. Diethylether behandelt und über Phosphorpentoxid getrocknet. Dabei wurden 0,32 g (88 % der Theorie) der Titelverbindung in Form dunkel gefärbter Kristalle erhalten, die an der Luft zerfließen.
- 1H-NMR (DMSO-d₆):: 2,10 - 2,80 (m, 4H); 4,35 (d, 1H); 4,56 (d, 1H); 5,03 (dd, 1H); 6,43 (d, 1 H); 6,60 (d, 1H); 7,03 (dd, 1H); 8,44 (d, 1H); 8,90 (s, 2H); 11,34 (s, 1H).

### b) 3-(7-Amino-4H-chinazolin-3-yl)piperidin-2,6-dion; Dihydrochlorid

Aus dem Produkt des Beispiels 7k ließ sich unter Anwendung der in Beispiel 9a beschriebenen Verfahrensweise analog die Titelverbindung in einer Ausbeute von 81 % der Theorie erhalten.
- 1H-NMR (DMSO-d₆):: 1,86 - 1,94 (m, 1H); 2,02 - 2,33 (m, 2H); 2,40 - 2,59 (m, 1H); 4,51 - 4,57 (m, 2H); 5,02 - 5,08 (m, 1H); 6,68 - 6,76 (m, 1H); 6,93 - 7,10 (m, 2H); 8,50 - 8,56 (m, 1H); 8,96 (s, 2H); 11,27 (s, 1H).

### Beispiel 10

### 3-(7-Fluor-4H-chinazolin-3-yl)1-piperidin-1-ylmethyl-piperidin-2,5-dion

Eine Suspension von 2,61 g des Produkts aus Beispiel 7e in 50 ml Ethanol wurde mit 1 ml wässriger Formaldehydlösung (37 %) und 0,8 ml Piperidin versetzt. Die Mischung wurde 1 Stunde bei 50°C gerührt, dann im Vakuum weitgehend vom Lösungsmittel befreit. Beim Versetzen des Rückstands mit Diethylether fielen (nach Abtrennen durch Filtration und Trocknen im Vakuum) 2,83 g (79 % der Theorie) der Titelverbindung in Form nahezu farbloser Kristalle an.
- 1H-NMR (DMSO-d₆):: 1,36 - 1,48 (m, 6H); 1,95 - 2,06 (m, 1H); 2,22 - 2,82 (m, 7H); 4,25 (d, 1H); 4,46 (d, 1H); 4,52 - 4,68 (m, 3H); 6,66 (dd, 1H); 6,81 (m, 1H); 6,90 - 6,95 (m, 1H); 7,11 (s, 1H).

### Untersuchung der immunmodulatorischen Wirksamkeit

Stimulation humaner Monozyten mit Lipopolysaccharid zur Sekretion von IL-10 und IL-12:
Humane Monozyten wurden aus peripheren Blut-mononucleären Zellen (PBMC), die mittels einer Ficoll-Dichtegradientenzentrifugation von heparinisiertem Vollblut gewonnen wurden, isoliert. Dazu wurden die PBMC mit einem monoklonalen Antikörper inkubiert, der gegen das Monozyten-spezifische Oberflächenmolekül CD14 gerichtet ist und an den superparamagnetische Microbeads (Miltenyi Biotech, Bergisch Gladbach) gekoppelt sind. Zur positiven Selektion der markierten Monozyten aus dem Zellgemisch der PBMC wurde die Gesamtzellsuspension auf eine Säule mit ferromagnetischer Trägermatrix aufgebracht und diese in ein Magnetfeld gestellt. Dadurch wurden die Zellen, die mit Microbeads beladen waren, an die Trägermatrix gebunden, unmarkierte Zellen passierten die Säule und wurden verworfen. Nach Herausnehmen der Matrix aus dem Magnetfeld wurden die Antikörper-beladenen Zellen durch Spülen der nun entmagnetisierten Säule mit Puffer eluiert. Die Reinheit dieser so erhaltenen CD14-positiven Monozytenpopulation beträgt etwa 95-98%. Diese Monozyten wurden in einer Dichte von 10⁶ Zellen/ml Kulturmedium (RPMI, supplementiert mit 10% foetalem Kälberserum) mit den in DMSO-gelösten Prüfsubstanzen für eine Stunde bei 37°C und 5% CO₂ inkubiert. Anschließend wurde 20 µg/ml LPS aus E. coli zugegeben. Nach 24 Stunden wurden zellfreie Kulturüberstände genommen und auf den Gehalt an IL-10 sowie IL-12 getestet.
Die Konzentration von IL-12 und IL-10 in den Zellkulturüberständen wurde mittels Sandwich-ELISAs unter Verwendung zweier anti-IL-12 bzw. IL-10 monoklonaler Antikörper (Biosource Europe, Fleurus, Belgien) bestimmt. Eine Referenzstandardkurve mit humanem IL-10 bzw. IL-12 wurde eingeschlossen. Das Detektionslimit des IL-12 ELISAs betrug 10 pg/ml, das des IL-10 ELISAs 15 pg/ml.

**Tabelle 1: Einfluß der Prüfsubstanzen im Vergleich zu Thalidomid auf die IL-12 und IL-10 Produktion von LPS-aktivierten Monozyten**

| Beispiel-Nr. | Hemmung der IL-12 Produktion | | Steigerung der IL-10 Produktion Maximal (%) |
|---|---|---|---|
| | Maximal (%) | IC50 (ng/ml) | |
| 1 | 99 | 5 | 360 |
| 3 | 98 | 10 | 230 |
| 7a | 98 | 1 | 365 |
| 7h | 97 | 6 | 316 |
| 7f | 98 | 0,8 | 420 |
| 7n | 84 | 42 | 263 |
| 7m | 89 | 50 | 202 |
| 7i | 96 | 36 | 211 |
| Thalidomid | 80 | 100 | 180 |

Die in 3-Position heterocyclisch substituierten Piperidin-2,6-dione der in Formel I beschriebenen Grundstruktur supprimierten in potenter Weise die IL-12 Produktion LPS-aktivierter Monozyten in einer konzentrationsabhängigen Weise. Interessanterweise wurde die IL-10 Produktion in demselben Konzentrationsbereich deutlich gesteigert. Die maximale IL-12 Hemmung sowie die IC50 Werte liegen deutlich über denen von Thalidomid. Die wirksamsten Verbindungen sind die, deren aromatischer Ring eine Chlor- oder Fluor-Substitution in Position 5 oder 7 aufweist bzw. unsubstituiert ist.

## Patentansprüche

1. In 3-Position heterocyclisch substituierte Piperidin-2,6-dione der allgemeinen Formel I, 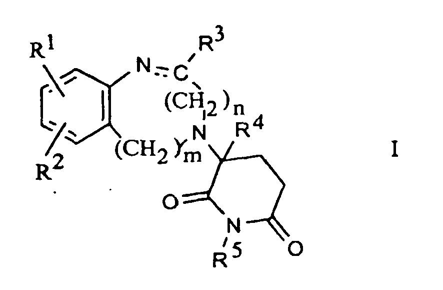 in der
R¹ und R², gleich oder verschieden voneinander, H, Br, Cl, F, CF₃, OH, NO₂, NH₂, N(CH₃)₂, C₁₋₃-Alkyl, C₁₋₃-Alkoxy, Phenyl oder zusammengenommen einen ankondensierten Benzolring bedeuten, wobei die Ringe gegebenenfalls mit R¹ und/oder R² substituiert und R¹ und R² wie oben definiert sind,
R³ für H, die Methylgruppe oder, für den Fall, daß eine C-N-Einfachbindung vorliegt, für OH, C₁₋₃-Alkoxy oder eine [O(CO)C₁₋₃-Alkyl]-Gruppe steht, oder zusammen mit dem C-Atom eine Carbonyl-Gruppe darstellt,
R⁴ H, F, CF₃ oder C₁₋₃-Alkyl bedeutet,
R⁵ für H, eine CH₂-OH-Gruppe oder einen Rest CH₂-NR⁶R⁷, in dem R⁶ und R⁷ gleich oder verschieden sind und eine Alkylgruppe mit 1-6 C-Atomen (geradkettig oder verzweigt) oder zusammen mit dem N-Atom einen Pyrrolidin-, Piperidin-, Hexamethylenimin- oder Morpholinring bedeuten, steht,
n 0 oder 1 und
m 0, 1 oder 2 bedeutet,
und deren reine Enantiomere oder nichtracemische Enantiomerengemische, Racemate, Diastereomere oder Diastereomerengemische in Form ihrer Basen oder Salze physiologisch verträglicher Säuren.

2. Verbindungen nach Anspruch 1, **dadurch gekennzeichnet, dass** R¹ und R², gleich oder verschieden voneinander, H, Br, Cl, F, CF₃, NO₂, NH₂, C₁₋₃-Alkyl, C₁₋₃-Alkoxy, oder zusammengenommen einen ankondensierten Benzolring bedeuten, R³ für H oder OH, R⁴ für H oder eine Methylgruppe und R⁵ für H oder einen Rest CH₂-NR⁶R⁷ steht, in dem R⁶ und R⁷ zusammen mit dem N-Atom einen Piperidinring bedeutet, und n = 0 und m = 1 oder 2 ist.

3. Verbindungen nach Anspruch 1 oder 2, **dadurch gekennzeichnet, dass** R¹ und R², gleich oder verschieden voneinander, H, Cl, F, CH₃ oder NO₂ bedeuten, R³, R⁴ und R⁵ Wasserstoff bedeuten, n = 0 und m = 1 ist und eine C=N-Doppelbindung vorliegt.

4. Verbindungen nach Anspruch 3, **dadurch gekennzeichnet, dass** sich die Reste R¹ und R² in Position 5 oder 7 befinden.

5. Verbindungen nach Anspruch 1:
3-(7-Chlor-4H-chinazolin-3-yl)piperidin-2,6-dion und das entsprechende Hydrobromid
3-(6-Chlor-4H-chinazolin-3-yl)piperidin-2,6-dion und das entsprechende Hydrobromid
3-(4H-Chinazolin-3-yl)piperidin-2,6-dion und das entsprechende Hydrobromid oder Hydrochlorid
3-(2-Hydroxy-1,4-dihydro-2H-chinazolin-3-yl)piperidin-2,6-dion
3-(6-Methoxy-4H-chinazolin-3-yl)piperidin-2,6-dion und das entsprechende Hydrochlorid
3-(5-Chlor-4H-chinazolin-3-yl)piperidin-2,6-dion
3-(8-Chlor-4H-chinazolin-3-yl)piperidin-2,6-dion
3-(6-Fluor-4H-chinazolin-3-yl)piperidin-2,6-dion
3-(8-Methoxy-4H-chinazolin-3-yl)piperidin-2,6-dion und das entsprechende Hydrochlorid
3-(7-Fluor-4H-chinazolin-3-yl)piperidin-2,6-dion und das entsprechende Hydrochlorid
3-(4H-Benzo[g]chinazolin-3-yl)piperidin-2,6-dion
3-(5-Fluor-4H-chinazolin-3-yl)piperidin-2,6-dion
3-(5-Nitro-4H-chinazolin-3-yl)piperidin-2,6-dion
3-(7-Trifluormethyl)-4H-chinazolin-3-yl)piperidin-2,6-dion
3-(7-Nitro-4H-chinazolin-3-yl)piperidin-2,6-dion
3-(8-Brom-6-methyl-4H-chinazolin-3-yl)piperidin-2,6-dion
3-(5-Methyl-4H-chinazolin-3-yl)piperidin-2,6-dion
3-(6,7-Difluor-4H-chinazolin-3-yl)piperidin-2,6-dion
3-Methyl-3-(4H-chinazolin-3-yl)piperidin-2,6-dion
3-(4,5-Dihydrobenzo[d][1,3]diazepin-3-yl)piperidin-2,6-dion
3-(5-Amino-4H-chinazolin-3-yl)piperidin-2,6-dion und das entsprechende Dihydrochlorid
3-(7-Amino-4H-chinazolin-3-yl)piperidin-2,6-dion und das entsprechende Dihydrochlorid
3-(7-Fluor-4H-chinazolin-3-yl)1-piperidin-1-ylmethyl-piperidin-2,6-dion.

6. Verfahren zur Herstellung von in 3-Position heterocyclisch substituierten Piperidin-2,6-dionen der allgemeinen Formel I nach Anspruch 1, bei dem zunächst eine Aminoverbindung der allgemeinen Formel II 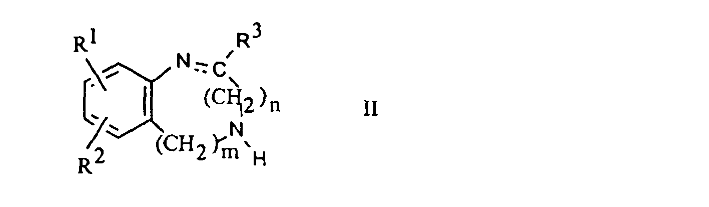 mit einem 3-Brompiperidin-2,6-dionderivat der allgemeinen Formel III 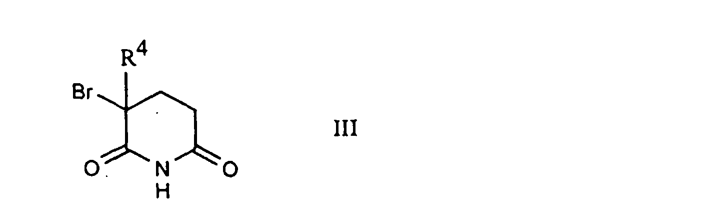 zu einer Verbindung der allgemeinen Formel IA alkyliert wird, 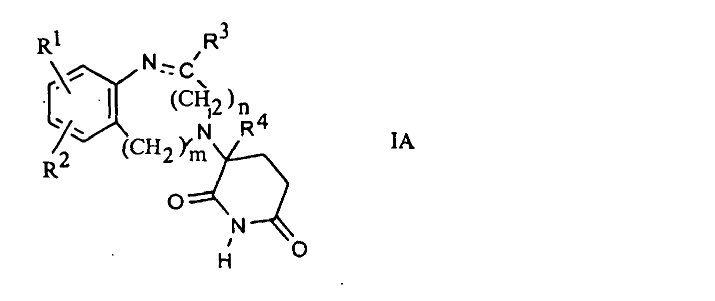 wobei in den Verbindungen IA, II und III die Reste R¹ bis R⁴ wie oben definiert sind, und anschließend, wenn R⁵ nicht für Wasserstoff stehen soll, dieser Rest durch Umsetzung mit Formaldehyd, gegebenenfalls zusammen mit einem Amin der allgemeinen Formel HNR⁶R⁷, in der R⁶ und R⁷ wie oben definiert sind, eingeführt wird und wenn in der Verbindung der Formel IA der Rest R⁴ für Wasserstoff steht, dieser für die Herstellung von weiteren erfindungsgemäßen Verbindungen mit R⁴ = F, CF₃ oder C₁₋₃-Alkyl durch an sich bekannte Alkylierungs- oder Halogenierungsreaktionen gegen die vorstehend genannten R⁴-Substituenten ausgetauscht wird.

7. Verfahren zur Herstellung von in 3-Position heterocyclisch substituierten Piperidin-2,6-dionen der allgemeinen Formel I nach Anspruch 1, wobei man eine Aminoverbindung der oben genannten Formel II zunächst mit einem 3-Brompiperidin-2-onderivat der Formel IV 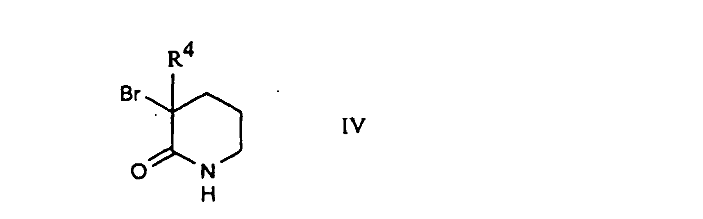 zu einer Verbindung der allgemeinen Formel IB alkyliert, 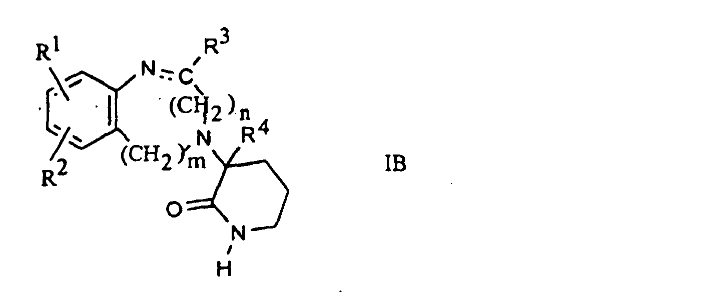 diese, vorzugsweise mit m-Chlor-perbenzoesäure oder Ruthenium (IV)-oxid/Natriumperiodat, zu einer Verbindung der oben genannten Formel IA oxidiert und gegebenenfalls andere Reste R⁴ und/oder R⁵ wie oben (Anspruch 6) beschrieben einführt.

8. Verfahren zur Herstellung von in 3-Position heterocyclisch substituierten Piperidin-2,6-dionen der allgemeinen Formel I nach Anspruch 1, in der m = 1 und n = 0 bedeuten und R³ für H oder OH steht, wobei man ein Formamidderivat der allgemeinen Formel VI, 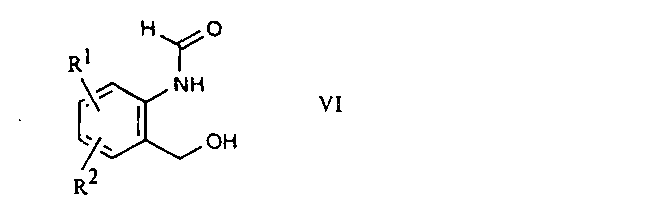 in dem R¹ und R² wie oben definiert sind, und das durch selektive N-Formylierung des entsprechenden 2-Aminobenzylalkohols oder durch selektive O-Entformylierung des N,O-Bisformylderivats zugänglich ist, zunächst in an sich bekannter Weise zu einem Benzaldehyd der allgemeinen Formel VII oxidiert, 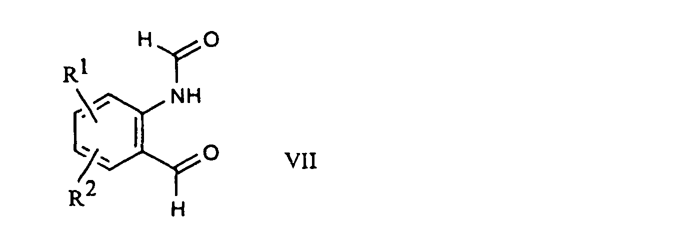 der durch reduktive Aminierung mit Glutamin unter Einsatz von komplexen Borhydriden in Verbindungen der allgemeinen Formel VIII übergeführt wird, 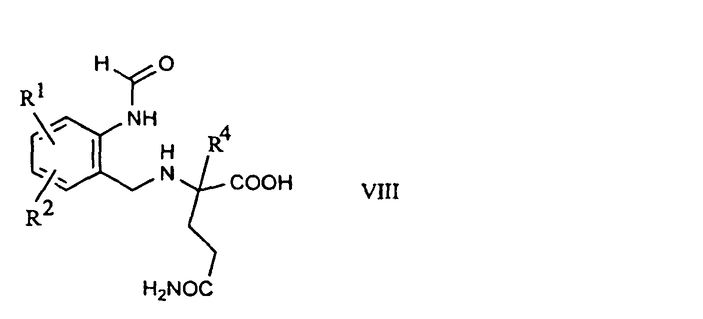 welche in Gegenwart aktivierender Reagenzien, vorzugsweise von N,N'-Carbonyldiimidazol, zu Glutarimiden der allgemeinen Formel IX cyclisiert werden, vorzugsweise nach vorherigem Schutz der Aminfunktion durch eine Schutzgruppe, vorzugsweise die Benzyloxycarbonylgruppe, die anschließend wieder abgespalten wird, 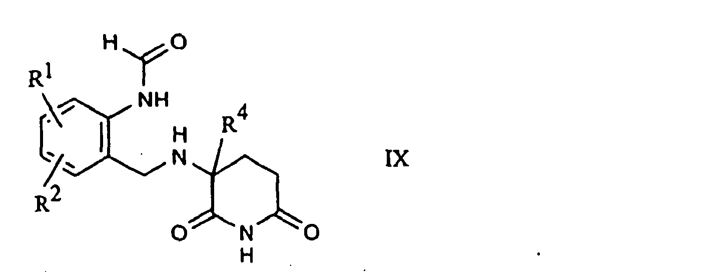 wobei
A. die Verbindungen der allgemeinen Formel IX in protischen Lösungsmitteln unter Säurekatalyse zu Verbindungen der allgemeinen Formel I, in der R¹, R² und R⁴ wie oben definiert sind, m für 1, n für 0 steht, eine C=N-Doppelbindung vorliegt und R³ bzw. R⁵ ein Wasserstoffatom darstellen, welches im Falle, dass R⁵ ungleich Wasserstoff sein soll, wie oben beschrieben gegen die übrigen definitionsgemäßen Substituenten ausgetauscht wird, umgesetzt wird oder
B. die Verbindungen der allgemeinen Formel IX, in denen die Aminfunktion durch eine Schutzgruppe geschützt ist, durch hydrogenolytische Abspaltung dieser Schutzgruppe zu Verbindungen der allgemeinen Formel I, in der R¹, R² und R⁴ wie oben definiert sind, eine C-N-Einfachbindung vorliegt und R³ für die Hydroxygruppe steht, umgesetzt werden und gegebenenfalls weiter mit verdünnten Säuren in organischen Lösungsmitteln durch Wasserabspaltung in Verbindungen der allgemeinen Formel I mit C=N-Doppelbindung und mit R³ = Wasserstoff umgewandelt werden.

9. Verfahren zur Herstellung von in 3-Position heterocyclisch substituierten Piperidin-2,6-dionen der allgemeinen Formel I nach Anspruch 1, in der m = 1 und n = 0 bedeuten und R³ für H steht, bei dem zunächst Verbindungen der oben genannten allgemeinen Formel VII gemäß Anspruch 8 hergestellt und reduktiv aminiert werden, wonach das Reaktionsgemisch mit Säuren behandelt und zu Verbindungen der allgemeinen Formel X, in der R¹, R² und R⁴ wie oben definiert sind, 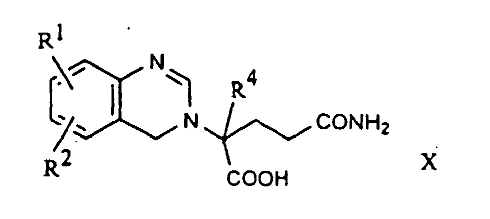 umgesetzt wird, die durch Cyclisierung in Verbindungen der allgemeinen Formel I mit einer C=N-Doppelbindung überführt werden und gegebenenfalls andere Reste R⁴ und/oder R⁵ wie oben (Anspruch 6) beschrieben eingeführt werden.

10. Verfahren nach Anspruch 9, **dadurch gekennzeichnet, dass** entsprechende Verbindungen der Formel I, jedoch mit m = 2, analog hergestellt werden.

11. Arzneimittel enthaltend als Wirkstoff wenigstens eine Verbindung der allgemeinen Formel I gemäß Anspruch 1 und/oder deren Enantiomere, Diastereomere, Basen oder Salze von physiologisch verträglichen Säuren.

12. Arzneimittel nach Anspruch 11 mit immunmodulatorischer Wirkung zur Behandlung und/oder Prophylaxe von Entzündungs- sowie Autoimmunerkrankungen und/oder hämatologisch-onkologischen Erkrankungen.

13. Verwendung wenigstens einer Verbindung der allgemeinen Formel I nach Anspruch 1 und/oder von deren Enantiomeren, Diastereomeren, Basen oder Salzen von physiologisch verträglichen Säuren zur Herstellung eines Arzneimittels zur Behandlung und/oder Prophylaxe von Entzündungs- sowie Autoimmunerkrankungen und/oder hämatologisch-onkologischen Erkrankungen.

## Claims

1. Piperidine-2,6-diones heterocyclically substituted in the 3-position of the general formula I 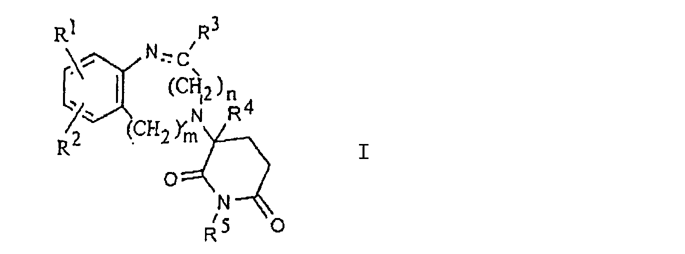 in which
R¹ and R², which are identical or different, denote H, Br, Cl, F, CF₃, OH, NO₂, NH₂, N(CH₃)₂, C₁₋₃-alkyl, C₁₋₃-alkoxy, phenyl, or together form an annelated benzene ring, in which the rings are optionally substituted by R¹ and/or R² and R¹ and R² are as defined above,
R³ denotes H, the methyl group or, in the case where a C-N single bond is present, denotes OH, C₁₋₃-alkoxy or an [O(CO)C₁₋₃-alkyl] group, or together with the C atom forms a carbonyl group,
R⁴ denotes H, F, CF₃ or C₁₋₃-alkyl,
R⁵ denotes H, a CH₂-OH group or a CH₂-NR⁶R⁷ radical, in which R⁶ and R⁷ are identical or different and denote an alkyl group with 1 to 6 C atoms (straight-chain or branched) or together with the N atom denote a pyrrolidine, piperidine, hexamethyleneimine or morpholine ring,
n is 0 or 1 and
m is 0, 1 or 2,
and their pure enantiomers or non-racemic enantiomer mixtures, racemates, diastereomers or diastereomer mixtures, in the form of their bases or salts of physiologically compatible acids.

2. Compounds according to claim 1, **characterised in that** R¹ and R², which are identical or different, denote H, Br, Cl, F, CF₃, NO₂, NH₂, C₁₋₃-alkyl, C₁₋₃-alkoxy, or together form an annelated benzene ring, R³ denotes H or OH, R⁴ denotes H or a methyl group and R⁵ denotes H or a CH₂-NR⁶R⁷ radical, in which R⁶ and R⁷ together with the N atom form a piperidine ring, and n = 0 and m = 1 or 2.

3. Compounds according to claim 1 or 2, **characterised in that** R¹ and R², which are identical or different from one another, denote H, Cl, F, CH₃ or NO₂, R³, R⁴ and R⁵ denote hydrogen, n = 0 and m = 1, and a C = N double bond is present.

4. Compounds according to claim 3, **characterised in that** the radicals R¹ and R² are in the 5-position or 7-position.

5. Compounds according to claim 1:
3-(7-chloro-4H-quinazolin-3-yl)piperidine-2,6-dione and the corresponding hydrobromide
3-(6-chloro-4H-quinazolin-3-yl)piperidine-2,6-dione and the corresponding hydrobromide
3-(4H-quinazolin-3-yl)piperidine-2,6-dione and the corresponding hydrobromide or hydrochloride
3-(2-hydroxy-1,4-dihydro-2H-quinazolin-3-yl)piperidine-2,6-dione
3-(6-methoxy-4H-quinazolin-3-yl)piperidine-2,6-dione and the corresponding hydrochloride
3-(5-chloro-4H-quinazolin-3-yl)piperidine-2,6-dione
3-(8-chloro-4H-quinazolin-3-yl)piperidine-2,6-dione
3-(6-fluoro-4H-quinazolin-3-yl)piperidine-2,6-dione
3-(8-methoxy-4H-quinazolin-3-yl)piperidine-2,6-dione and the corresponding hydrochloride
3-(7-fluoro-4H-quinazolin-3-yl)piperidine-2,6-dione and the corresponding hydrochloride
3-(4H-benzo[g]quinazolin-3-yl)piperidine-2,6-dione
3-(5-fluoro-4H-quinazolin-3-yl)piperidine-2,6-dione
3-(5-nitro-4H-quinazolin-3-yl)piperidine-2,6-dione
3-(7-trifluoromethyl)-4H-quinazolin-3-yl)piperidine-2,6-dione
3-(7-nitro-4H-quinazolin-3-yl)piperidine-2,6-dione
3-(8-bromo-6-methyl-4H-quinazolin-3-yl)piperidine-2,6-dione
3-(5-methyl-4H-quinazolin-3-yl)piperidine-2,6-dione
3-(6,7-difluoro-4H-quinazolin-3-y])piperidine-2,6-dione
3-methyl-3-(4H-quinazolin-3-yl)piperidine-2,6-dione
3-(4,5-dihydrobenzo[d][1,3]diazepin-3-yl)piperidiné-2,6-dione
3-(5-amino-4H-quinazolin-3-yl)piperidine-2,6-dione and the corresponding dihydrochloride
3-(7-amino-4H-quinazolin-3-yl)piperidine-2,6-dione and the corresponding dihydrochloride
3-(7-fluoro-4H-quinazolin-3-yl)1-piperidine-1-ylmethylpiperidine-2,6-dione.

6. Process for the production of piperidine-2,6-diones heterocyclically substituted in the 3-position of the general formula I according to claim 1, in which first of all an amino compound of the general formula II 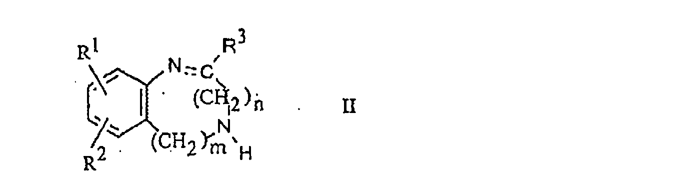 is alkylated with a 3-bromopiperidine-2,6-dione derivative of the general formula III 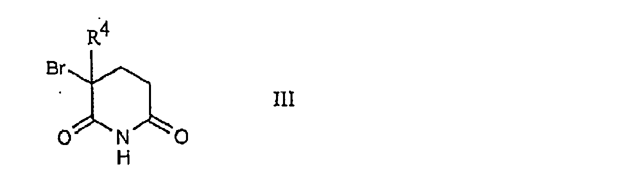 to form a compound of the general formula IA 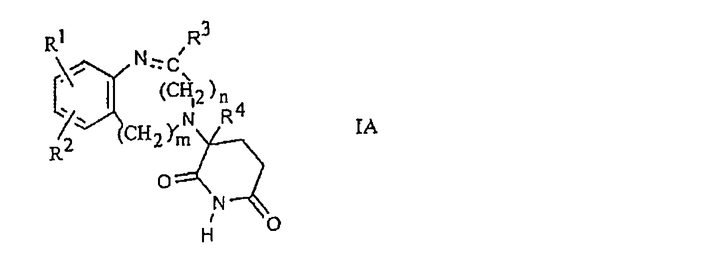 wherein in the compounds IA, II and III the radicals R¹ to R⁴ are defined as above, and then, if R⁵ is not intended to denote hydrogen, this radical is introduced by reaction with formaldehyde, optionally together with an amine of the general formula HNR⁶R⁷, in which R⁶ and R⁷ are as defined above, and if in the compound of the formula IA the radical R⁴ denotes hydrogen, then in order to produce further compounds according to the invention where R⁴ = F, CF₃ or C₁₋₃-alkyl, this is replaced by the aforementioned R⁴ substituents by means of alkylation or halogenation reactions known *per se.*

7. Process for the production of piperidine-2,6-diones heterocyclically substituted in the 3-position of the general formula I according to claim 1, wherein an amino compound of the aforementioned formula II is first of all alkylated with a 3-bromopiperidine-2-one derivative of the formula IV 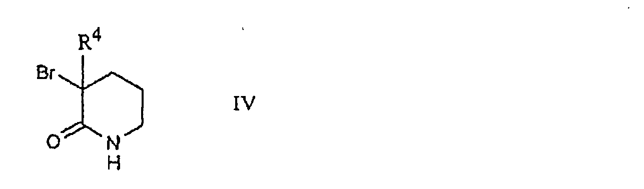 to form a compound of the general formula IB 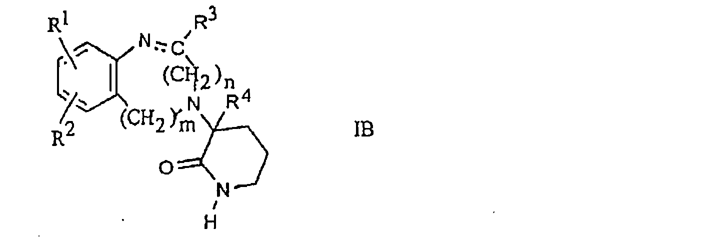 and this is oxidised, preferably with m-chloroperbenzoic acid or ruthenium (IV) oxide/sodium periodate, to form a compound of the aforementioned formula IA, and optionally other radicals R⁴ and/or R⁵ are introduced as described above (claim 6).

8. Process for the production of piperidine-2,6-diones heterocyclically substituted in the 3-position of the general formula I according to claim 1, in which m = 1 and n = 0 and R³ denotes H or OH, in which a formamide derivative of the general formula VI 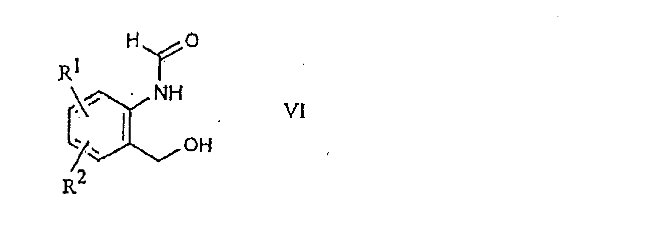 in which R¹ and R² are as defined above and which is accessible by selective N-formylation of the corresponding 2-aminobenzyl alcohol or by selective O-deformylation of the N,O-bisformyl derivative, is first of all oxidised in a manner known per se to form a benzaldehyde of the general formula VII 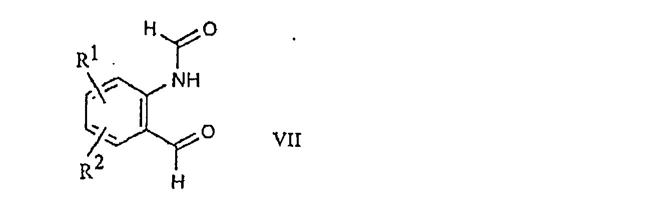 which is converted by reductive amination with glutamine using complex boron hydrides into compounds of the general formula VIII, 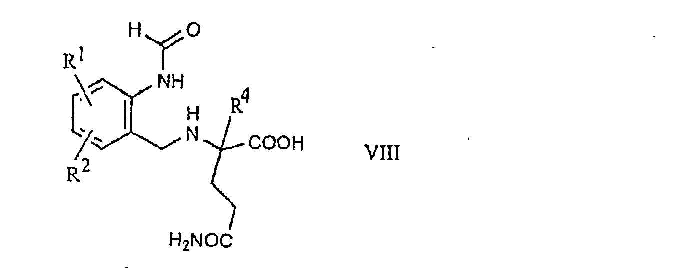 which are cyclised in the presence of activating reagents, preferably in the presence of N,N'-carbonyl diimidazole, to form glutarimides of the general formula IX, preferably after prior protection of the amine function by a protective group, preferably the benzyloxycarbonyl group, which is then split off again, 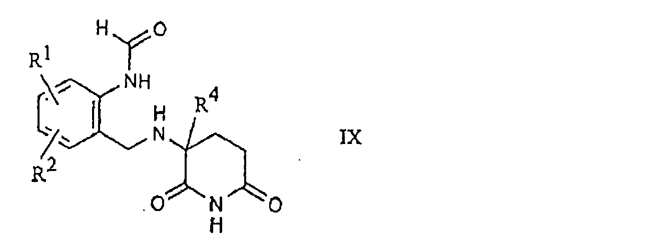 wherein
**A**. The compounds of the general formula IX are converted in protic solvents under acid catalysis to form compounds of the general formula I in which R¹, R² and R⁴ are as defined above, m is 1, n is 0, a C=N double bond is present, and R³ and/or R⁵ denote a hydrogen atom, which in the case where R⁵ is not hydrogen is replaced as described above by the other substituents according to the invention,
or
**B**. The compounds of the general formula IX in which the amine function is protected by a protective group are converted by hydrogenolytic splitting off of this protective group to form compounds of the general formula I in which R¹, R² and R⁴ are as defined above, a C-N single bond is present and R³ denotes the hydroxy group, and are optionally converted further with dilute acids in organic solvents by splitting off water, into compounds of the general formula I with a C=N double bond and where R³ = hydrogen.

9. Process for the production of piperidine-2,6-diones heterocyclically substituted in the 3-position of the general formula I according to claim 1, in which m = 1 and n = 0 and R³ denotes H, in which first of all compounds of the aforementioned general formula VII are prepared according to claim 8 and are reductively aminated, following which the reaction mixture is treated with acids and is converted into compounds of the general formula X in which R¹, R² and R⁴ are as defined above, 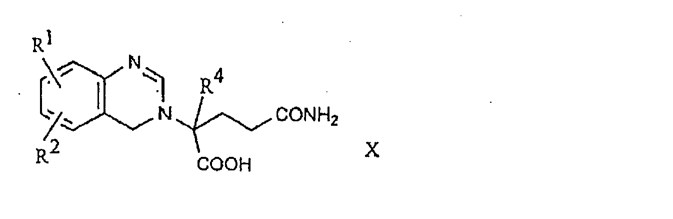 which are converted by cyclisation into compounds of the general formula I with a C=N double bond and optionally other radicals R⁴ and/or R⁵ as described above (claim 6) are introduced.

10. Process according to claim 9, **characterised in that** corresponding compounds of the formula I but where m = 2 are prepared in a similar manner.

11. Medicaments containing as active ingredient at least one compound of the general formula I according to claim 1 and/or their enantiomers, diastereomers, bases or salts of physiologically compatible acids.

12. Medicament according to claim 11 having an immunomodulating action for the treatment and/or prophylaxis of inflammatory and autoimmune diseases and/or haematological/oncological diseases.

13. Use of at least one compound of the general formula I according to claim 1 and/or their enantiomers, diastereomers, bases or salts of physiologically compatible acids for the production of a medicament for the treatment and/or prophylaxis of inflammatory and autoimmune diseases and/or haematological/oncological diseases.

## Revendications

1. Pipéridine-2,6-diones à substituant hétérocyclique en position 3, de formule générale I, 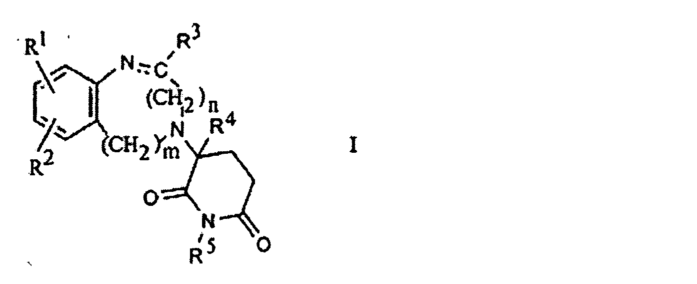 dans laquelle
R¹ et R², identiques ou différents l'un de l'autre, représentent H, Br, Cl, F, CF₃, OH, NO₂, NH₂, N (CH₃)₂, un reste alkyle en C₁ à C₃, alkoxy en C₁ à C₃, phényle, ou forment ensemble un noyau benzénique condensé, les noyaux étant éventuellement substitués avec R¹ et/ou R², et R¹ et R² étant tels que définis ci-dessus,
R³ représente H, le groupe méthyle ou bien, au cas où une liaison simple C-N est présente, un groupe OH, alkoxy en C₁ à C₃ ou un groupe [O(CO)-alkyle en C₁ à C₃], ou forme un groupe carbonyle conjointement avec l'atome C,
R⁴ représente H, F, CF₃ ou un reste alkyle en C₁ à C₃,
R⁵ représente H, un groupe CH₂-OH ou un reste CH₂-NR⁶R⁷ dans lequel R⁶ et R⁷ sont identiques ou différents et représentent un groupe alkyle ayant 1 à 6 atomes de carbone (linéaire ou ramifié) ou forment conjointement avec l'atome N un noyau pyrrolidine, pipéridine, hexaméthylène-imine ou morpholine,
n a la valeur 0 ou 1 et
m a la valeur 0, 1 ou 2,
et leurs énantiomères purs ou leurs mélanges d'énantiomères non racémiques, leurs racémates, leurs diastéréo-isomères ou leurs mélanges de diastéréo-isomères, sous forme de leurs bases ou sous forme de sels d'acides physiologiquement acceptables.

2. Composés suivant la revendication 1, **caractérisés en ce que** R¹ et R², identiques ou différents l'un de l'autre, représentent H, Br, Cl, F, CF₃, NO₂, NH₂, un reste alkyle en C₁ à C₃, alkoxy en C₁ à C₃, ou forment ensemble un noyau benzénique condensé, R³ représente H ou OH, R⁴ représente H ou un groupe méthyle et R⁵ représente H ou un reste CH₂-NR⁶R⁷ dans lequel R⁶ et R⁷ forment, conjointement avec l'atome N, un noyau pipéridine, n a la valeur 0 et m a la valeur 1 ou 2.

3. Composés suivant la revendication 1 ou 2, **caractérisés en ce que** R¹ et R², identiques ou différents l'un de l'autre, représentent H, Cl, F, CH₃ ou NO₂, R³, R⁴ et R⁵ représentent l'hydrogène, n est égal à 0 et m est égal à 1 et une double liaison C=N est présente.

4. Composés suivant la revendication 3, **caractérisés en ce que** les restes R¹ et R² se trouvent en position 5 ou 7.

5. Composés suivant la revendication 1, à savoir :
3-(7-chloro-4H-quinazoline-3-yl)-pipéridine-2,6-dione et le bromhydrate correspondant
3-(6-chloro-4H-quinazoline-3-yl)-pipéridine-2,6-dione et le bromhydrate correspondant
3-(4H-quinazoline-3-yl)-pipéridine-2,6-dione et le chlorhydrate ou bromhydrate correspondant
3-(2-hydroxy-1,4-dihydro-2H-quinazoline-3-yl)-pipéridine-2,6-dione
3-(6-méthoxy-4H-quinazoline-3-yl)-pipéridine-2,6-dione et le chlorhydrate correspondant
3-(5-chloro-4H-quinazoline-3-yl)-pipéridine-2,6-dione
3-(8-chloro-4H-quinazoline-3-yl)-pipéridine-2,6-dione
3-(6-fluoro-4H-quinazoline-3-yl)-pipéridine-2,6-dione
3-(8-méthoxy-4H-quinazoline-3-yl)-pipéridine-2,6-dione et le chlorhydrate correspondant
3-(7-fluoro-4H-quinazoline-3-yl)-pipéridine-2,6-dione et le chlorhydrate correspondant
3-(4H-benzo[g]quinazoline-3-yl)-pipéridine-2,6-dione
3-(5-fluoro-4H-quinazoline-3-yl)-pipéridine-2,6-dione
3-(5-nitro-4H-quinazoline-3-yl)-pipéridine-2,6-dione
3-(7-trifluorométhyl)-4H-quinazoline-3-yl)-pipéridine-2,6-dione
3-(7-nitro-4H-quinazoline-3-yl)-pipéridine-2,6-dione
3-(8-bromo-6-méthyl-4H-quinazoline-3-yl)-pipéridine-2,6-dione
3-(5-méthyl-4H-quinazoline-3-yl)-pipéridine-2,6-dione
3-(6,7-difluoro-4H-quinazoline-3-yl)-pipéridine-2,6-dione
3-méthyl-3-(4H-quinazoline-3-yl)-pipéridine-2,6-dione
3-(4,5-dihydrobenzo[d][1,3]diazépine-3-yl)-pipéridine-2,6-dione
3-(5-amino-4H-quinazoline-3-yl)-pipéridine-2,6-dione et le dichlorhydrate correspondant
3-(7-amino-4H-quinazoline-3-yl)-pipéridine-2,6-dione et le dichlorhydrate correspondant
3-(7-fluoro-4H-quinazoline-3-yl)-pipéridine-2,6-dione.

6. Procédé de production de pipéridine-2,6-dione à substituant hétérocyclique en position 3, de formule générale I suivant la revendication 1, dans lequel un composé aminé de formule générale II 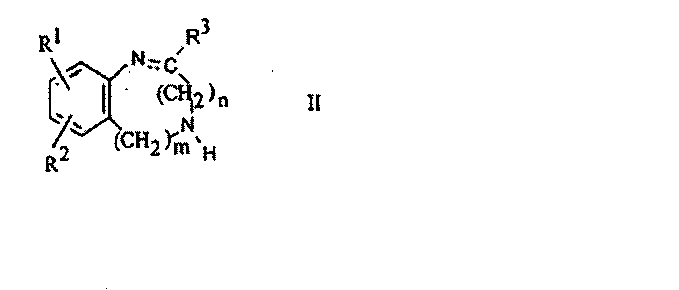 est tout d'abord alkylé avec un dérivé de 3-bromopipéridine-2,6-dione de formule générale III 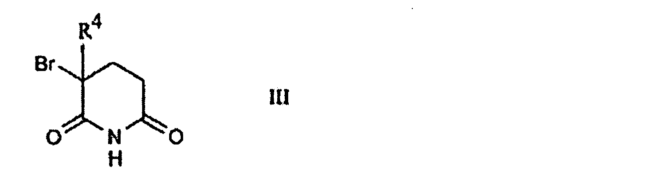 en un composé de formule générale IA 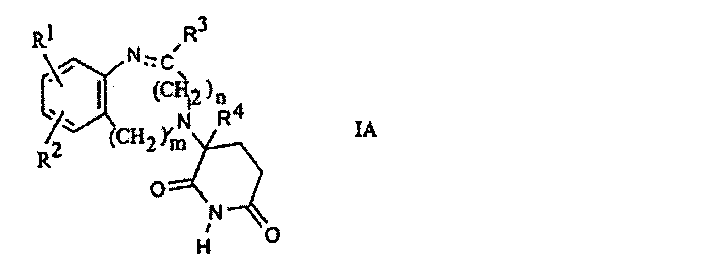 les restes R¹ à R⁴ dans les composés IA, II et III étant tels que définis ci-dessus, après quoi, lorsque R⁵ ne doit pas représenter l'hydrogène, ce reste est introduit par réaction avec le formaldéhyde, éventuellement conjointement avec une amine de formule générale HNR⁶R⁷, dans laquelle R⁶ et R⁷ sont tels que définis ci-dessus, et lorsque dans le composé de formule IA, le reste R⁴ représente l'hydrogène, ce reste est échangé, en vue de la préparation d'autres composés conformes à l'invention dans lesquels R⁴ représente F, CF₃ ou un reste alkyle en C₁ à C₃, contre les substituants R⁴ mentionnés ci-dessus, par des réactions connues d'alkylation ou d'halogénation.

7. Procédé de production de pipéridine-2,6-diones à substituant hétérocyclique en positon 3, de formule générale I suivant la revendication 1, dans lequel on alkyle tout d'abord un composé aminé de formule II mentionné ci-dessus avec un dérivé de 3-bromopipéridine-2-one de formule IV 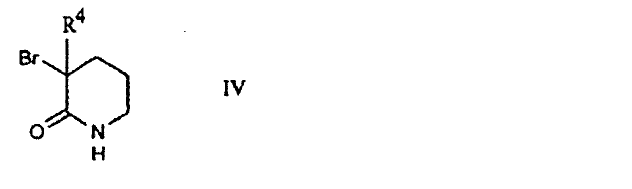 pour former un composé de formule générale IB 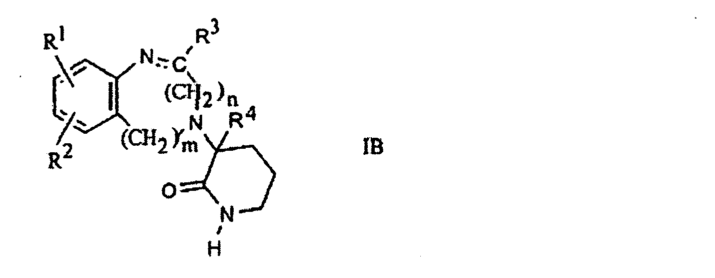 on oxyde ce composé, avantageusement avec l'acide m-chloroperbenzoïque ou le système oxyde de ruthénium (IV)/periodate de sodium, en un composé de formule I mentionné ci-dessus et, le cas échéant, on introduit d'autres restes R⁴ et/ou le reste R⁵ de la manière décrite ci-dessus dans la revendication 6.

8. Procédé de production de pipéridine-2,6-diones à substituant hétérocyclique en position 3, de formule générale I suivant la revendication 1, dans laquelle m est égal à 1 et n est égal à 0, et R³ représente H ou OH, dans lequel on oxyde tout d'abord d'une manière connue, un dérivé de formamide de formule générale VI, 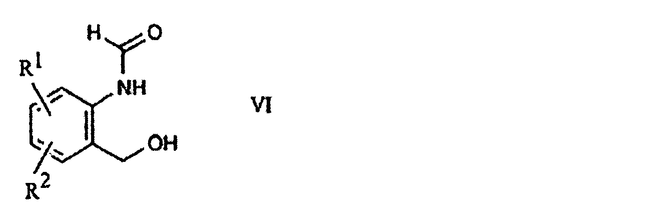 dans laquelle R¹ et R² sont tels que définis ci-dessus et que l'on peut obtenir par N-formylation sélective de l'alcool 2-aminobenzylique correspondant ou par O-déformylation sélective du dérivé N,O-bis-formylique, pour obtenir un benzaldéhyde de formule générale VII, 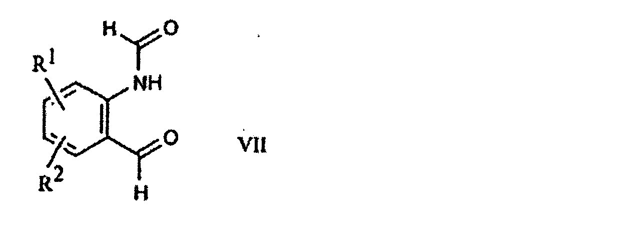 qu'on transforme par amination par voie de réduction avec la glutamine en utilisant des borohydrures complexes, en composés de formule générale VIII, 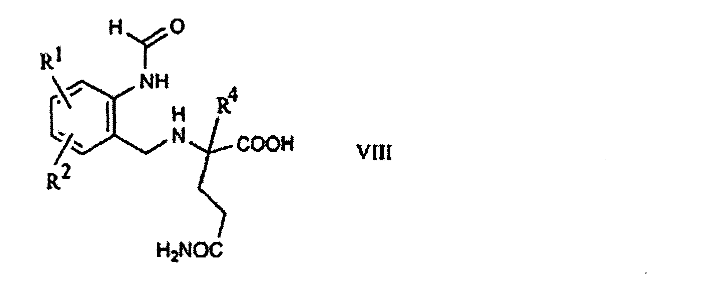 qu'on cyclise en présence de réactifs activateurs, avantageusement de N,N'-carbonyl-diimidazole, en glutarimides de formule générale IX, avantageusement après protection préalable de la fonction amine, par un groupe protecteur, avantageusement le groupe benzyloxycarbonyle, qu'on élimine ensuite 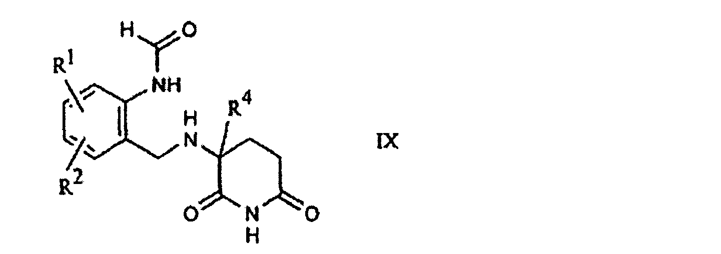 où
A. les composés de formule générale IX sont transformés dans des solvants protonés, avec catalyse par un acide, en composés de formule générale I dans laquelle R¹, R² et R⁴ sont tels que définis ci-dessus, m est égal à 1, n est égal à 0, une double liaison C=N est présente, et R³ et R⁵ représentent un atome d'hydrogène qui, au cas où R⁵ doit être différent de l'hydrogène, est remplacé comme décrit ci-dessus par les autres substituants conformes à la définition
ou bien
B. Les composés de formule générale IX dans lesquels la fonction amine est protégée par un groupe protecteur sont transformés par élimination par hydrogénolyse de ce groupe protecteur en composés de formule générale I dans laquelle R¹, R² et R⁴ sont tels que définis ci-dessus, une liaison simple C-N est présente et R³ représente un groupe hydroxy, et, le cas échéant, encore transformés avec des acides dilués dans des solvants organiques, par élimination d'eau, en composés de formule générale I avec une double liaison générale I dans lesquels il existe une double liaison C=N et où R³ représente l'hydrogène.

9. Procédé de production de pipéridine-2,6-diones à substituant hétérocyclique en position 3, de formule générale I selon la revendication 1, dans laquelle m est égal à 1 et n est égal à 0 et R³ représente H, dans lequel, tout d'abord, des composés de formule générale VII mentionnés ci-dessus sont préparés conformément à la revendication 8 et aminés par voie de réduction, après quoi le mélange réactionnel est traité avec des acides et transformé en composés de formule générale X dans laquelle R¹, R² et R⁴ sont tels que définis ci-dessus, 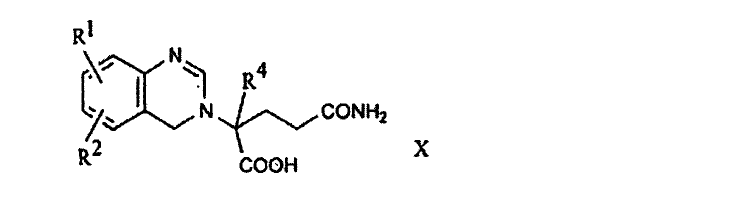 qui sont transformés par cyclisation en composés de formule générale I avec une double liaison C=N, et, le cas échéant, d'autres restes R⁴ et/ou R⁵ tels que définis ci-dessus (revendication 6) sont introduits.

10. Procédé suivant la revendication 9, **caractérisé en ce que** des composés correspondants de formule I sont préparés de façon analogue, toutefois avec m = 2.

11. Médicament contenant comme substance active au moins un composé de formule générale I suivant la revendication 1 et/ou ses énantiomères, diastéréo-isomères, bases ou sels d'acides physiologiquement acceptables.

12. Médicament suivant la revendication 11, à action immunomodulatrice, destiné au traitement et/ou à la prophylaxie de maladies inflammatoires ainsi que de maladies auto-immunes et/ou de maladies oncologiques de nature hématologique.

13. Utilisation d'au moins un composé de formule générale I suivant la revendication 1 et/ou de ses énantiomères, diastéréo-isomères, bases ou sels d'acides physiologiquement acceptables pour la préparation d'un médicament destiné au traitement et/ou à la prophylaxie de maladies inflammatoires ainsi que de maladies auto-immunes et/ou de maladies oncologiques de nature hématologique.
